# EUROPEAN PATENT SPECIFICATION

(11) **EP 3 845 155 B1**
(45) Date of publication and mention of the grant of the patent: **27.03.2024**
(21) Application number: 19855206.9
(22) Date of filing: 07.08.2019
(51) Int. Cl.: A61B 17/29, A61B 34/35, B25J 17/00, A61B 34/30, A61B 34/00

(54) **ROBOTIC SURGICAL APPARATUS**
CHIRURGISCHE ROBOTERVORRICHTUNG
APPAREIL CHIRURGICAL ROBOTISÉ

(30) Priority: 28.08.2018 JP 2018159336
(43) Date of publication of application: 07.07.2021
(73) Proprietor: Medicaroid Corporation, Hyogo 650-0047 (JP)
(72) Inventor: BETSUGI, SHOTA, Kobe-shi, Hyogo 650-0047 (JP); USUKI, Yu, Kobe-shi, Hyogo 650-0047 (JP)
(74) Representative: Müller Hoffmann & Partner
(86) International application number: PCT/JP2019/031064
(87) International publication number: WO 2020/044994

(56) References cited:
- WO-A1-2017/098266
- WO-A1-2017/098266
- JP-A- 2011 045 500
- JP-A- 2017 513 678
- US-A1- 2004 199 147
- US-A1- 2010 198 253
- US-B1- 6 394 998
- US-B1- 6 394 998

## Description

### TECHNICAL FIELD

The invention relates to a robotic surgical instrument and particularly relates to a robotic surgical instrument that includes a first support body supporting an end effector rotatably about a first shaft and a second support body supporting the first support body rotatably about a second shaft.

### BACKGROUND ART

Conventionally, there has been known a robotic surgical instrument and especially a robotic surgical instrument that includes a first support body supporting an end effector rotatably about a first shaft and a second support body supporting the first support body rotatably about a second shaft. Such a robotic surgical instrument is disclosed in U.S. Patent No. 6394998, for example.

U.S. Patent No. 6394998 discloses a surgical instrument that includes an end effector, a clevis (hereinafter, a first clevis) as a U-shaped coupling tool that supports the end effector pivotably about a pivot shaft (hereinafter, a first pivot shaft), and a clevis (hereinafter, a second clevis) that is provided on the side of a shaft and supports the first clevis pivotably about a pivot shaft (hereinafter, a second pivot shaft). The surgical instrument disclosed in U.S. Patent No. 6394998 includes a rotating cable for rotating the end effector about the axial direction of the first pivot shaft. The surgical instrument disclosed in U.S. Patent No. 6394998 also includes a pulley portion rotatably supported by the first pivot shaft and a pulley group including plural pulleys rotatable about rotation axes parallel to the second pivot shaft. Groove widths of the plural pulleys of the pulley group are the same as each other and the plural pulleys are provided on end sides in the rotation axes.

WO2017/098266A1 discloses a robotic surgical instrument comprising: a shaft; an articulation at a distal end of the shaft for articulating an end effector, the articulation driveable by a pair of driving elements; and an instrument interface at a proximal end of the shaft, the instrument interface comprising an instrument interface element for driving the pair of driving elements, the instrument interface element displaceable over a first displacement range, the instrument interface element comprising a body receivable in a drive assembly interface element of a robot arm when the robotic surgical instrument engages with the surgical robot arm, the drive assembly interface element displaceable over a second displacement range, the length of the body in the displaceable direction being greater than one of the maximum travel of the body over the first displacement range and the maximum travel of the drive assembly interface element over the second displacement range.

US6394998B1 discloses surgical tools or instruments for use in minimally invasive telesurgical applications. The instruments typically include a base whereby the instrument is removably mountable on a robotically controlled articulated arm. An elongate shaft extends from the base. A working end of the shaft is disposed at an end of the shaft remote from the base. A wrist member is pivotally mounted on the working end. At least one end effector element mounting formation is pivotally mounted on an opposed end of the wrist member. A plurality of elongate elements, e.g., cables, extend from the end effector element mounting formation and the wrist member to cause selective angular displacement of the wrist member and end effector mounting formation in response to selective pulling of the elongate elements.

US2004/199147A1 discloses a manipulator for medical use comprises a surgical tool portion, a grip portion provided on this surgical tool portion, and a driver portion for driving the grip portion provided on the surgical tool portion. A connection portion of the surgical tool is provided between the surgical tool portion and the driver portion, and a surgical tool can be separated from and connected to, between the driver portion and this surgical tool side connection portion. The surgical tool portion has two pieces of rotating members, being disposed opposing to each other, a driving means for driving one of those rotating members to revolute to the other thereof, but not rotate by itself, and a means for keeping a distance between rotation centers of those two pieces of rotating members to be constant.

US2010/198253A1 discloses a medical manipulator including a wire movable in opposite directions, a driven wire having both ends thereof connected to the wire, an end effector of a distal-end working unit, a transmitting member, a crescent driven member integral with the transmitting member, and a return pulley. The transmitting member, the crescent driven member, and the return pulley are successively arranged in this order from the proximal end of the medical manipulator. When the driven wire is moved in opposite directions, the transmitting member also moves in opposite directions. At this time, the crescent driven member moves toward the return pulley, and the proximal-end portion of the return pulley enters a cavity of the crescent driven member.

### PRIOR ART DOCUMENTS

### PATENT LITERATURE

Patent Document 1: U.S Patent No. 6394998

### SUMMARY OF THE INVENTION

### PROBLEMS TO BE SOLVED BY THE INVENTION

However, in the surgical instrument disclosed in U.S. Patent No. 6394998, when the rotating cable that is passed through the pulley group arranged at the end in the direction of the rotation axis parallel to the second axis and is drawn into the shaft, the rotating cable may come into contact with the inner wall of the shaft. In order to solve this problem, the insertion hole of the second clevis on the shaft side into which the rotating cable is drawn is needed to be arranged on the side of the center (the center in the direction of the rotation axis) of the second clevis.

However, when the insertion hole of the second clevis on the shaft side is arranged on the side of the center (the center in the rotation axis direction) of the second clevis as described above, the rotating cable is suspended diagonally in the pulley groove of the pulley in the pulley group that is arranged closest to the insertion hole. In this case, the rotating cable being obliquely suspended comes into contact with a side portion of the pulley groove on the side of the insertion hole, and thus the load applied to the rotating cable (elongate element) is increased. As a result, there is a problem that it is difficult to smoothly guide the rotating cable (elongated element).

The invention provides a robotic surgical instrument capable of smoothly guiding an elongate element. The invention is defined in appended independent claim 1. Further embodiments are defined in appended dependent claims.

### MEANS FOR SOLVING THE PROBLEM

A robotic surgical instrument according to one aspect of the invention comprises: an end effector; a first support body that rotatably supports the end effector about a first shaft, an elongate element for driving the end effector; a second support body that rotatably supports the first support body about a second shaft; a first pulley that is rotatably provided about a third shaft at a position between the first and second shafts and on which an elongate element is suspended; a second pulley rotatably provided about the second shaft and on which the elongate element is suspended; and a shaft having an extending direction and connected to the second support body at an end portion of the shaft in the extending direction, wherein a groove width of a shaft-side pulley, which is arranged at a position closest to the shaft among a plurality of pulleys including at least the first pulley and the second pulley and to which the elongate element is suspended, is greater than groove widths of the other pulleys among the plurality of pulleys.

As described above, the robotic surgical instrument according to the aspect of the invention includes the first pulley on which the elongate element is suspended, and the second pulley rotatably provided about the second shaft and on which the elongate element is suspended. Further, the groove width of the shaft-side pulley arranged at the position closest to the shaft side among the plurality of pulleys including at least the first pulley and the second pulley is greater than the groove widths of the other pulleys. With this configuration, even if the elongate element is suspended obliquely in the shaft-side pulley, the elongate element is less likely to come into contact with a side portion of the pulley groove of the shaft-side pulley on the first support body side, so that the extending direction of the elongate element is less likely to be changed. As a result, the shaft-side pulley can suppress an increase in the load applied to the elongate element, so as to smoothly guide the elongate element.

### EFFECTS OF THE INVENTION

According to the invention, the elongate element can be smoothly guided.

### BRIEF DESCRIPTION OF DRAWINGS

FIG. 1 is a diagram illustrating an overview of a robotic surgical system according to a first embodiment.
FIG. 2 is a block diagram illustrating a view of a control-related configuration of the robotic surgical system according to a first embodiment.
FIG. 3 is a diagram illustrating a perspective view of a state where a surgical instrument is attached to a robot arm via an adaptor according to a first embodiment.
FIG. 4 is a diagram illustrating a perspective view of the surgical instrument according to a first embodiment.
FIG. 5 is a diagram illustrating a schematic view of an elongate element of the surgical instrument according to a first embodiment.
FIG. 6A is a diagram illustrating a schematic view of a hook of the surgical instrument according to a first embodiment; and FIG. 6B is a diagram illustrating a schematic view of a first pulley portion of the surgical instrument according to a first embodiment.
FIG. 7 is a diagram illustrating a perspective view of the surgical instrument according to a first embodiment as seen from the X1 side.
FIG. 8 is a diagram illustrating a perspective view of a second support body of the surgical instrument according to a first embodiment.
FIG. 9 is a diagram illustrating a perspective view of a silicone seal of the surgical instrument according to a first embodiment.
FIG. 10 is a diagram illustrating a perspective view of a state where a connection portion of a shaft is inserted in the second support body of the surgical instrument according to a first embodiment.
FIG. 11 is a diagram illustrating a schematic view of a state where a first elongate element is wound around a first pulley group, a second pulley group, and a third pulley group of the surgical instrument according to a first embodiment.
FIG. 12A is a diagram illustrating a perspective view of a first (third) guide pulley of the surgical instrument according to a first embodiment; and FIG. 12B is a diagram illustrating a cross section view of the first (third) guide pulley of the surgical instrument according to a first embodiment.
FIG. 13A is a diagram illustrating a perspective view of a fifth guide pulley of the surgical instrument according to a first embodiment; and FIG. 13B is a diagram illustrating a cross section view of the fifth guide pulley of the surgical instrument according to a first embodiment.
FIG. 14 is a diagram illustrating a side view of the first pulley group, the second pulley group, and the third pulley group of the surgical instrument according to a first embodiment, as seen from the X1 side.
FIG. 15 is a diagram illustrating a side view of the first pulley group, the second pulley group, and the third pulley group of the surgical instrument according to a first embodiment, as seen from the Y1 side.
FIG. 16 is a diagram illustrating a schematic view of a state where the first elongate element is suspended on the first pulley group, the second pulley group, and the third pulley group and passes through insertion holes of the silicone seal of the surgical instrument according to a first embodiment.
FIG. 17A is a diagram illustrating a side view of the third guide pulley of the surgical instrument according to a first embodiment, as seen from the Y1 side; and FIG. 17B is a diagram illustrating a side view of the fifth guide pulley of the surgical instrument according to a first embodiment, as seen from the Y1 side.
FIG. 18 is a diagram illustrating a side view of a state where the first elongate element is wound around the first pulley group, the second pulley group, and the third pulley group of the surgical instrument according to a first modification, as seen from the X1 side.
FIG. 19 is a diagram illustrating a perspective view of a surgical instrument according to a second embodiment.
FIG. 20A is a diagram illustrating a schematic view of a first jaw of the surgical instrument according to a first embodiment; and FIG. 20B is a diagram illustrating a schematic view of a first pulley portion of the surgical instrument according to a first embodiment.
FIG. 21A is a diagram illustrating a schematic view of a second jaw of the surgical instrument according to a first embodiment; and FIG. 21B is a diagram illustrating a schematic view of a second pulley portion of the surgical instrument according to a first embodiment.
FIG. 22 is a diagram illustrating a perspective view of a second support body of a surgical instrument according to a second embodiment.
FIG. 23 is a diagram illustrating a perspective view of a silicone seal of the surgical instrument according to a second embodiment.
FIG. 24 is a diagram illustrating a schematic view of a state where a first elongate element and a second elongate element are wound around a first pulley group and a second pulley group of the surgical instrument according to a second embodiment.
FIG. 25A is a diagram illustrating a perspective view of a first inner pulley portion of the surgical instrument according to a second embodiment; and FIG. 25B is a diagram illustrating a cross section view of the first inner pulley potion of the surgical instrument according to a second embodiment.
FIG. 26A is a diagram illustrating a perspective view of a third inner pulley portion of the surgical instrument according to a second embodiment; and FIG. 26B is a diagram illustrating a cross section view of the third inner pulley potion.
FIG. 27 is a diagram illustrating a side view of the first pulley group and the second pulley group of the surgical instrument according to a second embodiment, as seen from the X1 side.
FIG. 28 is a diagram illustrating a side view of the first pulley group and the second pulley group of the surgical instrument according to a second embodiment, as seen from the Y1 side.
FIG. 29 is a diagram illustrating a schematic view of a state where a first elongate element and a second elongate element are suspended on the first pulley group and the second pulley group and pass through insertion holes of the silicone seal of the surgical instrument according to a second embodiment.
FIG. 30A is a diagram illustrating a side view of a first inner pulley portion of a surgical instrument according to a second modification, as seen from the Y1 side; and FIG. 30B is a diagram illustrating a side view of the third inner pulley portion of the surgical instrument according to a second modification, as seen from the Y1 side.
FIG. 31 is a diagram illustrating a side view of a state where a first elongate element and a second elongate element are suspended on a first pulley group and a second pulley group of the surgical instrument according to a second modification, as seen from the X1 side.

### DETAILED DESCRIPTION

Descriptions are provided hereinbelow for one or more embodiments based on the drawings. In the respective drawings referenced herein, the same constituents are designated by the same reference numerals and duplicate explanation concerning the same constituents is omitted. All of the drawings are provided to illustrate the respective examples only.

### [First Embodiment]

### (Configuration of Robotic Surgical System)

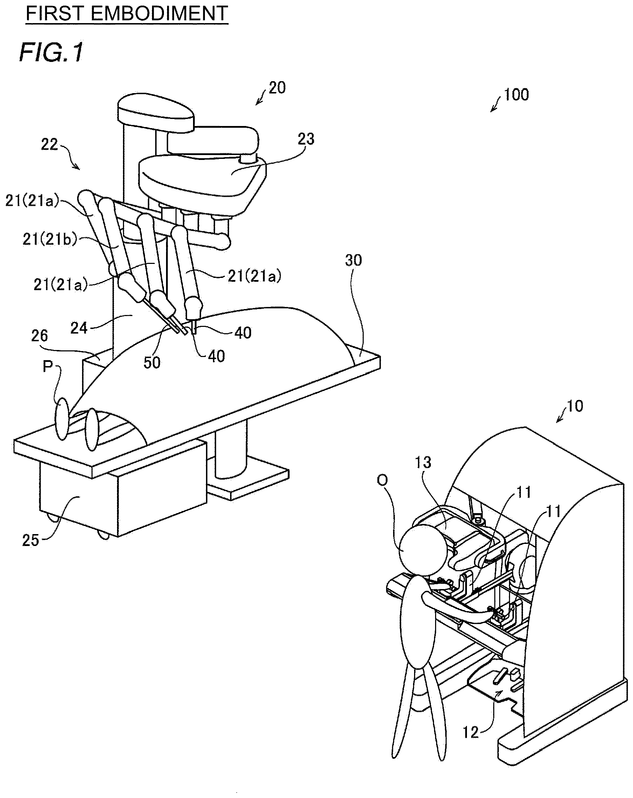

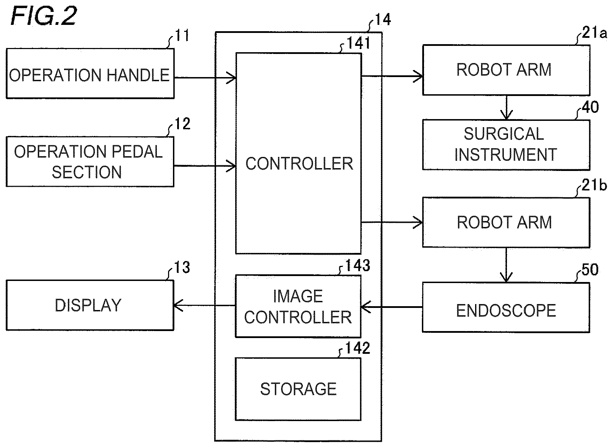

The configuration of a robotic surgical system 100 according to a first embodiment is described with reference to FIGS. 1 and 2.

As illustrated in FIG. 1, the robotic surgical system 100 includes a remote control apparatus 10 and a patient-side apparatus 20. The remote control apparatus 10 is provided to remotely control medical equipment provided for the patient-side apparatus 20. When an operator O, as a surgeon, inputs an action mode instruction to be executed by the patient-side apparatus 20, to the remote control apparatus 10, the remote control apparatus 10 transmits the action mode instruction to the patient-side apparatus 20 through a controller 26. In response to the action mode instruction transmitted from the remote control apparatus 10, the patient-side apparatus 20 operates medical equipment such as surgical instruments 40, an endoscope 50, and the like, attached to robot arms 21. This allows for minimally invasive surgery.

The patient-side apparatus 20 constitutes an interface to perform a surgery for a patient P. The patient-side apparatus 20 is positioned beside an operation table 30 on which the patient P is laid. The patient-side apparatus 20 includes plural robot arms 21. One of the robot arms 21 (21b) holds an endoscope 50 while the other robot arms 21 (21a) hold the surgical instruments 40. The robot arms 21 are commonly supported by a platform 23. Each of the plural robot arms 21 includes plural joints. Each joint includes a driver provided with a servo-motor and a position detector such as an encoder. The robot arms 21 are configured so that the medical equipment attached to each robot arm 21 is controlled by a driving signal given through the controller 26 and performs a desired movement.

The platform 23 is supported by a positioner 22 placed on the floor of an operation room. The positioner 22 includes a column 24 and a base 25. The column 24 includes an elevating shaft adjustable in the vertical direction. The base 25 includes wheels and is movable on the floor surface.

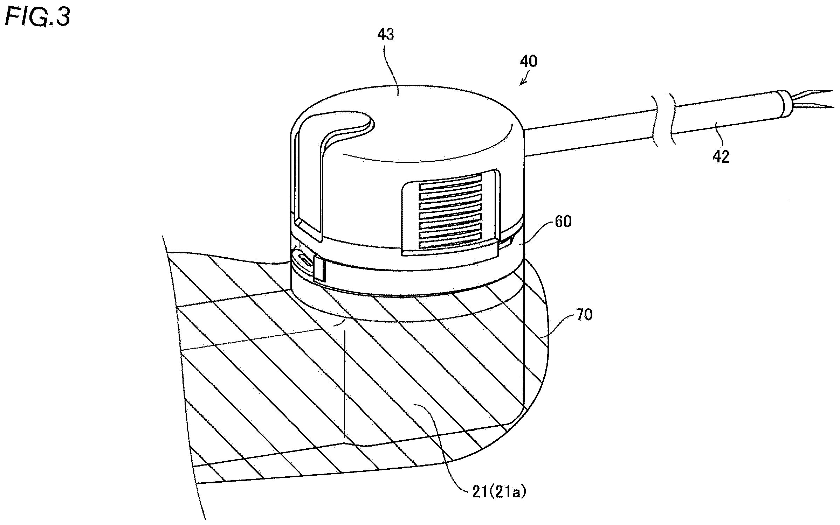

The surgical instruments 40 as the medical equipment is detachably attached to the distal ends of the robot arms 21a. As illustrated in FIG. 3, each surgical instrument 40 includes: a housing 43, which is attached to the robot arm 21a; an elongate shaft 42; and an end effector 41, which is provided at the distal end portion of the shaft 42. The end effector 41 is grasping forceps, scissors, a hook, a high-frequency knife, a snare wire, a clamp, or a stapler, for example. The end effector 41 is not limited to those and can be various types of treatment tools. In surgeries using the patient-side apparatus 20, the robot arms 21a introduce the surgical instruments 40 into the body of the patient P through a cannula (trocar) placed on the body surface of the patient P. The end effectors 41 of the surgical instruments 40 are then located near a surgery site.

To the distal end of the robot arm 21b, the endoscope 50 as the medical equipment is detachably attached. The endoscope 50 captures an image in a body cavity of the patient P. The captured image is outputted to the remote control apparatus 10. The endoscope 50 is a 3D endoscope capable of capturing a three-dimensional image or a 2D endoscope. In surgeries using the patient-side apparatus 20, the robot arm 21b introduces the endoscope 50 into the body of the patient P through a trocar placed on the body surface of the patient P. The endoscope 50 is then located near the surgery site.

The remote control apparatus 10 constitutes the interface with the operator O. The remote control apparatus 10 is an apparatus that allows the operator O to operate medical equipment attached to the robot arms 21. Specifically, the remote control apparatus 10 is configured to transmit action mode instructions which are inputted by the operator O and are to be executed by the surgical instruments 40 and endoscope 50, to the patient-side apparatus 20 through the controller 26. The remote control apparatus 10 is installed beside the operation table 30 so that the operator O can see the condition of the patient P very well while operating the remote control apparatus 10, for example. The remote control apparatus 10 may be configured to transmit action mode instructions wirelessly and installed in a room different from the operation room where the operation table 30 is installed.

The action modes to be executed by the surgical instruments 40 include modes of actions to be taken by each surgical instrument 40 (a series of positions and postures) and actions to be executed by the function of each surgical instrument 40. When the surgical instrument 40 is a pair of grasping forceps, for example, the action modes to be executed by the surgical instrument 40 include roll and pitch positions of the wrist of the end effector 41 and actions to open and close the jaws. When the surgical instrument 40 is a high-frequency knife, the action modes to be executed by the surgical instrument 40 include vibration of the high-frequency knife, specifically, supply of current to the high-frequency knife. When the surgical instrument 40 is a snare wire, the action modes to be executed by the surgical instrument 40 include a capturing action and an action to release the captured object. Further the action modes may include an action to supply current to a bipolar or monopolar instrument to burn off the surgery site.

The action modes to be executed by the endoscope 50 include the position and posture of the tip of the endoscope 50 and setting of the zoom magnification, for example.

As illustrated in FIGS. 1 and 2, the remote control apparatus 10 includes operation handles 11, an operation pedal section 12, a display 13, and a control apparatus 14.

The operation handles 11 are provided in order to remotely operate medical equipment attached to the robot arms 21. Specifically, the operation handles 11 accept operations by the operator O for operating the medical equipment (the surgical instruments 40 and endoscope 50). The operation handles 11 include two operation handles 11 arranged side by side in the horizontal direction. One of the two operation handles 11 is operated by the right hand of the operator O while the other operation handle 11 is operated by the left hand of the operator O.

The operation handles 11 extend from the rear side of the remote control apparatus 10 toward the front side. The operation handles 11 are configured to move in a predetermined three-dimensional operation region. Specifically, the operation handles 11 are configured so as to move up and down, right and left, and forward and rearward.

The remote control apparatus 10 and patient-side apparatus 20 constitute a master-slave system in terms of controlling movement of the robot arms 21a and robot arm 21b. The operation handles 11 constitute an operating part on the master side in the master-slave system, and the robot arms 21a and 21b holding the medical equipment constitute an operating part on the slave side. When the operator O operates the operation handles 11, the movement of one of the robot arms 21a and 21b is controlled so that the distal end portion (the end effector 41 of the surgical instrument 40) of the robot arm 21a or the distal end portion (the endoscope 50) of the robot arm 21b moves following the movement of the operation handles 11.

The patient-side apparatus 20 controls the movement of the robot arms 21a in accordance with the set motion scaling ratio. When the motion scaling ratio is set to 1/2, for example, the end effectors 41 of the surgical instruments 40 move a half of the movement distance of the operation handles 11. This allows for precise fine surgery.

The operation pedal section 12 includes plural pedals to execute medical equipment-related functions. The plural pedals include a coagulation pedal, a cutting pedal, a camera pedal, and a clutch pedal. The plural pedals are operated by a foot of the operator O.

The coagulation pedal enables the surgical instrument 40 to coagulate the surgery site. Specifically, when the coagulation pedal is operated, voltage for coagulation is applied to the surgical instrument 40 to coagulate the surgery site. The cutting pedal enables the surgical instrument 40 to cut the surgery site. Specifically, the cutting pedal is operated to apply voltage for cutting to the surgical instrument 40 and cut the surgery site.

The camera pedal is used to control the position and orientation of the endoscope 50 that captures images within the body cavity. Specifically, the camera pedal enables operation of the endoscope 50 by the operation handles 11. That is, the position and orientation of the endoscope 50 are controllable by the operation handles 11 while the camera pedal is being pressed. The endoscope 50 is controlled by using both of the right and left operation handles 11, for example. Specifically, when the operator O rotates the right and left operation handles 11 about the middle point between the right and left operation handles 11, the endoscope 50 is rotated. When the operator O presses the right and left operation handles 11 together, the endoscope 50 goes forward into the body cavity. When the operator O pulls the right and left operation handles 11 together, the endoscope 50 goes back. When the operator O moves the right and left operation handles 11 together up, down, right, or left, the endoscope 50 moves up, down, right, or left, respectively.

The clutch pedal is used to temporarily disconnect operation-related connection between the operation handles 11 and the robot arms 21 to stop movement of the surgical instruments 40. Specifically, when the clutch pedal is being pressed, the robot arms 21 of the patient-side apparatus 20 do not work even if the operation handles 11 are operated. For example, when the operation handles 11 are operated and moved to the edge of the range of movement, the operator O operates the clutch pedal to temporarily disconnect the operation-related connection and then returns the operation handles 11 to the center of the range of movement. When the operator O stops operating the clutch pedal, the operation handles 11 are again connected to the robot arms 21. The operator O restarts the operation for the operation handles 11 around the center thereof.

The display 13 or a display unit is configured to display images captured by the endoscope 50. The display section 13 includes a scope type display or a non-scope type display. The scope type display is a display that the operator O looks into. The non-scope type display is a display like an open-type display that includes a flat screen and the operator O is able to see without looking into, such as normal displays for personal computers.

When the scope type display is attached, the scope type display displays 3D images captured by the endoscope 50 attached to the robot arm 21b of the patient-side apparatus 20. When the non-scope type display is attached, the non-scope type display also displays 3D images captured by the endoscope 50 provided for the patient-side apparatus 20. The non-scope type display may display 2D images captured by the endoscope 50 provided for the patient-side apparatus 20.

As illustrated in FIG. 2, the control apparatus 14 includes a controller 141, a storage 142, and an image controller 143, for example. The controller 141 includes a calculator such as a CPU. The storage 142 includes a memory, such as a ROM and a RAM. The control apparatus 14 may be composed of a single controller performing centralized control or may be composed of plural controllers that perform decentralized control in cooperation with each other. The controller 141 determines whether an action mode instruction inputted by the operation handles 11 is to be executed by the robot arm 21a or to be executed by the endoscope 50, depending on the state of the operation pedal section 12. When determining that the action mode instruction inputted by the operation handles 11 is to be executed by any one of the surgical instruments 40, the controller 141 transmits the action mode instruction to the corresponding robot arm 21a. The robot arm 21a is thereby driven for controlling movement of the surgical instrument 40 attached to the robot arm 21a.

When determining that the action mode instruction inputted by the operation handles 11 is to be executed by the endoscope 50, the controller 141 transmits the action mode instruction to the robot arm 21b. The robot arm 21b is thereby driven for control of movement of the endoscope 50 attached to the robot arm 21b.

The storage 142 stores control programs corresponding to the types of the surgical instrument 40, for example. The controller 141 reads the stored control programs according to the types of the attached surgical instruments 40. The action mode instructions from the operation handles 11 and/or the operation pedal section 12 of the remote control apparatus 10 thereby cause the respective surgical instruments 40 to perform proper movements.

The image controller 143 transmits images acquired by the endoscope 50 to the display 13. The image controller 143 performs processing and alternations for the images when needed.

### (Configurations of Surgical Instrument)

With reference to FIGS. 3 to 16, the configuration of the surgical instrument 40 according to a first embodiment is described.

As illustrated in FIG. 3, the robot arm 21 is used in a clean area and is covered with a drape 70. In operation rooms, clean technique is used in order to prevent surgical incision sites and the medical equipment from being contaminated by pathogen, foreign matters, or the like. The clean technique defines a clean area and a contaminated area, which is other than the clean area. The surgery sites are located in the clean area. Members of the surgical team, including the operator O, make sure that only sterile objects are placed in the clean area during surgery and perform sterilization for an object which is to be moved to the clean area from the contaminated area. Similarly, when the members of the surgical team including the operator O place their hands in the contaminated area, the members sterilize their hands before directly touching objects located in the clean area. Instruments used in the clean area are sterilized or are covered with the sterile drape 70.

### (Surgical Instrument)

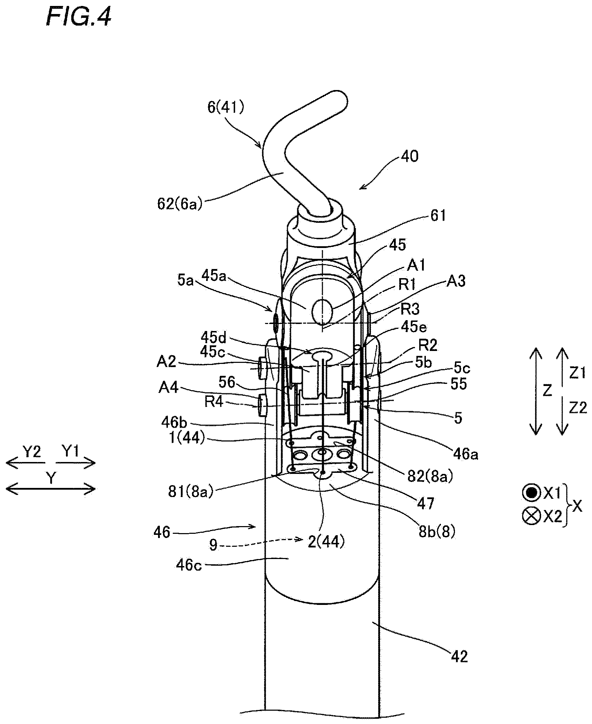

As illustrated in FIG. 4, the surgical instrument 40 is configured to operate the end effector 41 at a tip or a distal end portion of surgical instrument 40 by driving of elongate elements 44 that are driven by a (not-illustrated) drive mechanism in the robot arm 21 (see FIG. 3). The surgical instrument 40 is an example of a "robotic surgical instrument."

Specifically, the surgical instrument 40 includes the elongate elements 44, the above-described end effector 41, a first support body 45, a second support body 46, a silicone seal 47, a pulley group 5, and the shaft 42. The pulley group 5 includes a first pulley group 5a, a second pulley group 5b, and a third pulley group 5c. The pulley group 5 is an example of "a plurality of pulleys."

The first support body 45 rotatably supports the end effector 41 about a first shaft A1. In other words, the end effector 41 is attached to the first support body 45 so as to rotate about a rotation axis R1 of the first shaft A1. The second support body 46 rotatably supports the first support body 45 about a second shaft A2. In other words, the first support body 45 is attached to the second support body 46 so as to rotate about a rotation axis R2 of the second shaft A2. Note that the rotation axis R2 of the second shaft A2 is an example of a "rotation center axis of another pulley." The second shaft A2 is an example of "another pulley shaft."

Note that a direction in which the rotation axis R1 of the first shaft A1 extends is the X direction. One side of the X direction is the X1 direction and the other side is the X2 direction. A direction in which the rotation axis R2 of the second shaft A2 extends is the Y direction. One side of the Y direction is the Y1 direction and the other side is the Y2 direction. The X direction and the Y direction are orthogonal to each other. A direction orthogonal to the X and Y directions is the Z direction. One side of the Z direction is the Z1 direction and the other side is the Z2 direction. The X direction is an example of an "axial direction of the first shaft." The Y direction is an example of an "axial direction of the second shaft."

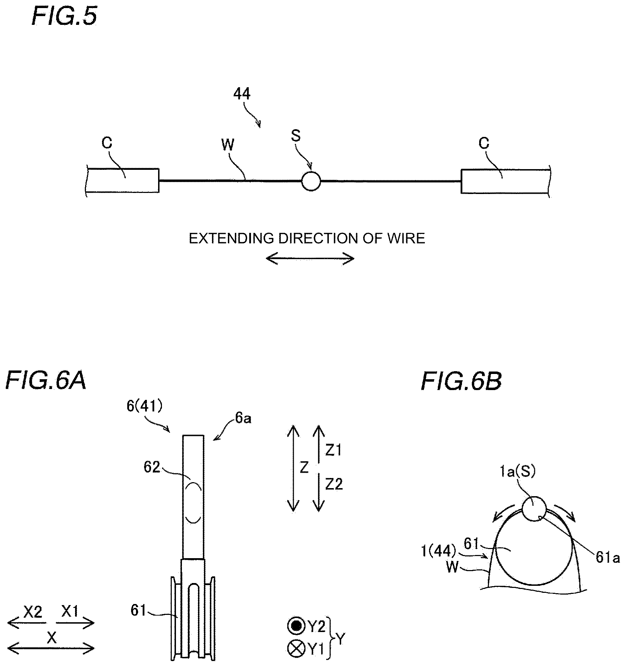

As illustrated in FIG. 5, each elongate element 44 includes a wire W, an attachment S fixed to the wire W, and protection tubes C fixed to the wire W. The wire W is made of metal such as stainless or tungsten. The protection tubes C are formed of rigid tubes partially covering the wire W. The plural (two) protection tubes C are arranged on the wire W. The attachment S is made of metal such as stainless. The attachment S is formed in a spherical shape or a column shape. The attachment S is arranged between the protection tubes C on the wire W.

As illustrated in FIG. 4, the end effector 41 is configured to perform procedures of the surgery site on the patient P (see FIG. 1) based on the function of a type of the end effector 41. Specifically, the end effector 41 includes a single end effector member 6. The end effector member 6 is a hook 6a. The hook 6a is attached to the first support body 45.

As illustrated in FIGS. 6A and 6B, the hook 6a includes a first pulley portion 61 and a first procedure portion 62. The first pulley portion 61 is formed with a recess 61a engaged with an attachment S of an elongate element 44 (hereinafter, an attachment 1a of a first elongate element 1). The procedure portion 62 changes the orientation by rotation of the first pulley portion 61 in accordance with movement of the first elongate element 1. The attachment 1a of the first elongate element 1 is formed in a circular column shape.

### (First Support Body)

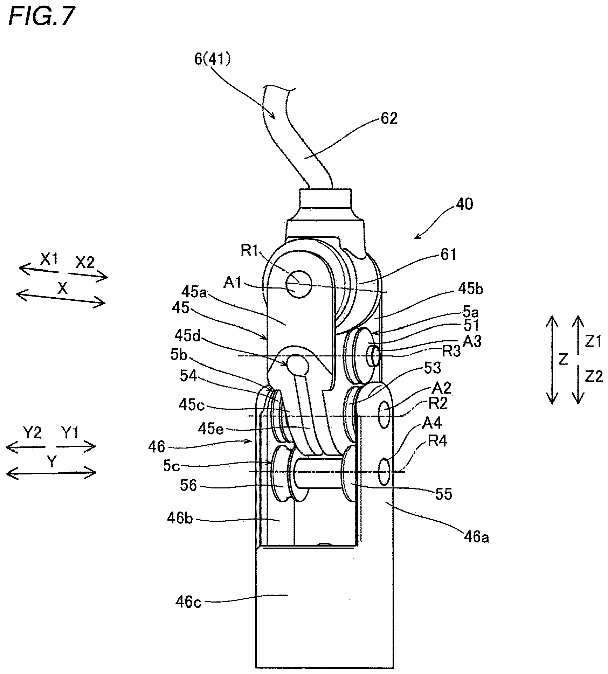

As illustrated in FIG. 7, the first support body 45 includes a first protrusion portion 45a, a second protrusion portion 45b, a second pulley portion 45c, and a through hole 45d. The first protrusion portion 45a protrudes in the Z1 direction from an end portion on the X1 side of the second pulley portion 45c. The first protrusion portion 45a supports an end portion on the X1 side of the first shaft A1. The second protrusion portion 45b protrudes in the Z1 direction from an end portion on the X2 side of the second pulley portion 45c. The second protrusion portion 45b supports an end portion on the X2 side of the first shaft A1. The second pulley portion 45c is rotatably supported by a second shaft A2. The second pulley portion 45c includes a pulley groove 45e formed along a circumferential direction of the second shaft A2. To the second pulley portion 45c, an elongate element 44 (hereinafter referred to as a second elongate element 2 (see FIG. 4)) is suspended, in order to rotate the first support body 45. The through hole 45d has a size through which the second elongate element 2 can be inserted. The through hole 45d is configured to linearly pass through the first support body 45 along the X direction.

### (Second Support Body)

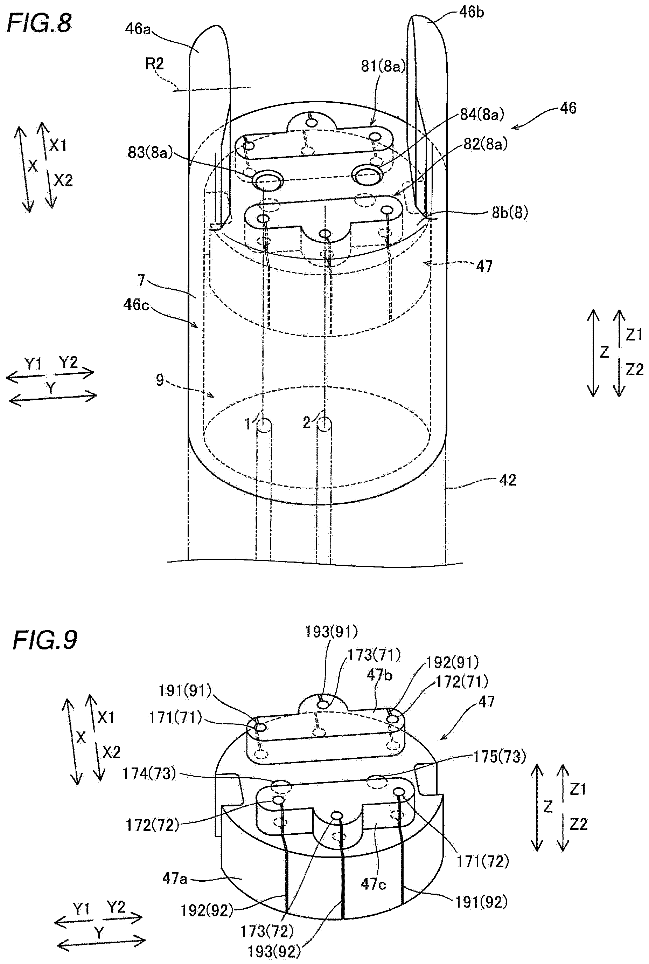

As illustrated in FIG. 8, the second support body 46 includes a third protrusion portion 46a, a fourth protrusion portion 46b, and a connection base portion 46c. The third protrusion portion 46a protrudes in the Z1 direction from an end portion on the Y1 side of the connection base portion 46c. The third protrusion portion 46a supports an end portion on the Y1 side of the second shaft A2. The fourth protrusion portion 46b protrudes in the Z1 direction from an end portion on the Y2 side of the connection base portion 46c. The fourth protrusion portion 46b supports an end portion on the Y2 side of the second shaft A2. An end portion on the Z2 side of the connection base portion 46c is connected to an end portion on the Z1 side of the shaft 42.

The connection base portion 46c is formed in a substantially cylindrical shape. The connection base portion 46c includes a side surface portion 7 and an end surface portion 8. The side surface portion 7 is provided along a circumferential direction around an axis extending in the Z direction. The end surface portion 8 is provided in an end portion of the side surface portion 7 on the other side opposite to the shaft 42 (an end portion on the Z1 side). The connection base portion 46c includes an inner space 9 surrounded by the side surface portion 7 and the end surface portion 8. The inner space 9 of the connection base portion 46c is opened toward the Z2 direction.

The end surface portion 8 includes a partition wall 8b including communication holes 8a passing through the partition wall 8b in the extending direction of the shaft 42 (the Z direction). The partition wall 8b is configured to separate the inner space 9 and the outer space of the connection base portion 46c.

The partition wall 8b is formed with the communication holes 8a passing through the partition wall 8b in the extending direction of the shaft 42 (the Z direction). That is, the second support body 46 includes the communication holes 8a that penetrates, in the extending direction of the shaft 42 (the Z direction), the partition wall 8b on the opposite side of the shaft 42. The communication holes 8a allow communication between the inner space 9 of the connection base portion 46c and the outer space of the connection base portion 46c. In the partition wall 8b, a communication hole 8a located on the X1 side (hereinafter, a first communication hole 81) and a communication hole 8a located on the X2 side (hereinafter, a second communication hole 82) are formed. Each of the first communication hole 81 and the second communication hole 82 is formed in a substantial T-shape as seen from the Z1 direction.

In the partition wall 8b, a communication hole 8a located on the Y1 side (hereinafter, a third communication hole 83) and a communication hole 8a located on the Y2 side (hereinafter, a fourth communication hole 84) are also formed. Each of the third communication hole 83 and the fourth communication hole 84 is formed in a substantially circular shape as seen from the Z1 direction.

### (Silicone Seal)

As illustrated in FIGS. 8 and 9, a silicone seal 47 is inserted in the inner space 9 of the second support body 46. The silicone seal 47 is arranged in a Z1 side end portion of the inner space 9 of the second support body 46. The silicone seal 47 is configured to seal (close) the first communication hole 81 and the second communication hole 82. The silicone seal 47 is an example of a "seal member."

The silicone seal 47 includes a first seal portion 47a, a second seal portion 47b, and a third seal portion 47c. The first seal portion 47a is formed in an H-shape as seen from the Z1 direction. The first seal portion 47a is in close contact with the surface of the end surface portion 8 of the second support body 46 on the X2 side. On the first seal portion 47a, the second seal portion 47b is arranged on the X1 side, and the third seal portion 47c is arranged on the X2 side.

The second seal portion 47b is formed correspondingly to the first communication hole 81 of the second support body 46. Specifically, the second seal portion 47b is formed in a substantial T-shape as seen from the Z1 direction correspondingly to the shape of the first communication hole 81 as seen from the Z1 direction. The third seal portion 47c is formed correspondingly to the second communication hole 82 of the second support body 46. Specifically, the third seal portion 47c is formed in a substantial T-shape as seen from the Z1 direction correspondingly to the shape of the second communication hole 82 as seen from the Z1 direction.

The silicone seal 47 includes a first insertion hole group 71, a second insertion hole group 72, and a third insertion hole group 73.

The first insertion hole group 71 and the second insertion hole group 72 each include a first insertion hole 171 through which the wire W of the first elongate element 1 is inserted, a second insertion hole 172 through which the wire W of the first elongate element 1 is inserted, and a third insertion hole 173 through which the wire W of the second elongate element 2 is inserted. The first insertion hole 171, the second insertion hole 172, and the third insertion hole 173 of the first insertion hole group 71 penetrate through the first seal portion 47a and the second seal portion 47b in the Z direction. The first insertion hole 171, the second insertion hole 172, and the third insertion hole 173 of the second insertion hole group 72 pass through the first seal portion 47a and the third seal portion 47c in the Z direction. Note that each of the first insertion hole 171 and the second insertion hole 172 is an example of an "insertion hole."

Consequently, the wire W of the first elongate element 1 can be easily moved while the silicone seal 47 prevents the entry of foreign material inside the second support body 46.

The third insertion hole group 73 includes a fourth insertion hole 174 and a fifth insertion hole 175 through which electric wires are inserted that supplies power to the end effector 41. The fourth insertion hole 174 and the fifth insertion hole 175 of the third insertion hole group 73 pass through only the first seal portion 47a in the Z direction.

The silicone seal 47 includes a first slit group 91 and a second slit group 92. The first slit group 91 includes a first slit 191 that is connected to the first insertion hole 171 of the first insertion hole group 71 and guides the wire W of the first elongate element 1 to the first insertion hole 171 of the first insertion hole group 71. The first slit group 91 includes a second slit 192 that is connected to the second insertion hole 172 of the first insertion hole group 71 and guides the wire W of the first elongate element 1 to the second insertion hole 172 of the first insertion hole group 71. The first slit group 91 includes a third slit 193 that is connected to the third insertion hole 173 of the first insertion hole group 71 and guides the wire W of the second elongate element 2 to the third insertion hole 173 of the first insertion hole group 71.

Similarly, the second slit group 92 also includes a first slit 191, a second slit 192, and a third slit 193 so as to correspond to the first insertion hole 171 and the second insertion hole 172 and the third insertion hole 173 of the second insertion hole group 72, respectively.

### <Shaft>

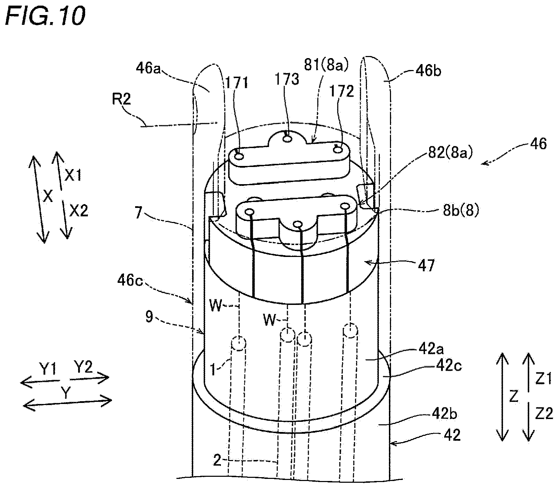

As illustrated in FIG. 10, the shaft 42 is formed in a cylindrical shape extending along the Z direction. The first elongate element 1 and the second elongate element 2 are housed in the space inside the shaft 42. The shaft 42 includes the cylindrical connection portion 42a and a cylindrical body portion 42b. The connection portion 42a protrudes in the Z1 direction from an end portion on the Z1 side of the body portion 42b. The body portion 42b extends in the Z direction. The shaft 42 includes a step 42c between the connection portion 42a and the body portion 42b. The step 42c of the shaft 42 comes into contact with an end portion on the Z2 side of the side surface portion 7 of the second support body 46.

As described above, the connection portion 42a is connected to the second support body 46. Specifically, the connection portion 42a is inserted in the inner space 9 of the second support body 46. In this state, the silicone seal 47 is compressed between the partition wall 8b and the connection portion 42a. This allows the silicone seal 47 to more tightly seal the first communication hole 81 and the second communication hole 82. Additionally, the silicone seal 47 can be more rigidly and closely attached to the wire W of the first elongate element 1 passing through the first insertion hole 171 and the wire W of the second elongate element 2 passing through the second insertion hole 172. Consequently, the sealing capability of the silicone seal 47 can be improved.

### (First Pulley Group and Second Pulley Group)

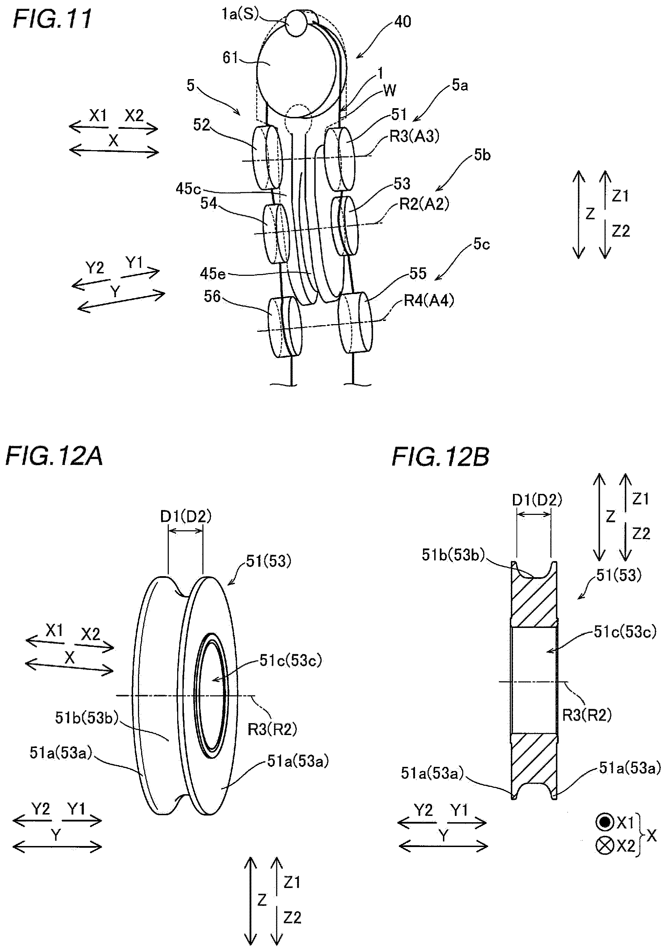

As illustrated in FIG. 11, the surgical instrument 40 includes a first pulley group 5a and a second pulley group 5b that guide the first elongate element 1 engaged with the first pulley portion 61.

The first pulley group 5a is rotatably provided about the third shaft A3 at a position between the first shaft A1 and the second shaft A2, and includes a first guide pulley portion 51 and a second guide pulley portion 52 that suspend the first elongate element 1. Here, the first guide pulley portion 51 and the second guide pulley portion 52 rotate about a rotation axis R3 of the third shaft A3, respectively. The first guide pulley portion 51 and the second guide pulley portion 52 are arranged at an end portion of the third shaft A3 on the Y1 side and an end portion of the third shaft A3 on the Y2 side, respectively. Since the first guide pulley portion 51 and the second guide pulley portion 52 have the same shape, only the first guide pulley portion 51 arranged at the end portion on the Y1 side will be described below. Note that the first guide pulley portion 51 and the second guide pulley portion 52 are examples of a "first pulley" and "another pulley." The third shaft A3 is an example of "another pulley shaft." The rotation axis R3 of the third shaft A3 is an example of a "rotation center axis of another pulley."

As illustrated in FIGS. 12A and 12B, the first guide pulley portion 51 is configured to rotate about the rotation axis R3 of the third shaft A3. Specifically, the first guide pulley portion 51 includes two disc portions 51a facing each other in the Y direction, a pulley groove 51b provided between the two disc portions 51a, and a shaft insertion hole 51c penetrating therethrough in the Y direction. Here, the pulley groove 51b of the first guide pulley portion 51 has a first predetermined groove width D1 in the Y direction. Note that the first predetermined groove width D1 is an example of a "groove width of another pulley." Further, the pulley groove 51b of the first guide pulley portion 51 is an example of a "groove of another pulley."

As illustrated in FIG. 11, the second pulley group 5b is rotatably provided about the second shaft A2, and has a third guide pulley portion 53 and a fourth guide pulley portion 54 that suspend the first elongate element 1. Here, the third guide pulley portion 53 and the fourth guide pulley portion 54 have the same configuration as the first guide pulley portion 51 and the second guide pulley portion 52 described above, respectively. The third guide pulley portion 53 and the fourth guide pulley portion 54 are examples of a "second pulley" and "another pulley."

That is, the third guide pulley portion 53 and the fourth guide pulley portion 54 are arranged at an end portion of the second shaft A2 on the Y1 side and an end portion of the second shaft A2 on the Y2 side, respectively. Since the third guide pulley portion 53 and the fourth guide pulley portion 54 have the same shape, only the third guide pulley portion 53 arranged at the end portion on the Y1 side will be described below.

As illustrated in FIGS. 12A and 12B, the third guide pulley portion 53 is configured to rotate about the rotation axis R2 of the second shaft A2. Specifically, the third guide pulley portion 53 includes two disc portions 53a facing each other in the Y direction, a pulley groove 53b provided between the two disc portions 53a, and a shaft insertion hole 53c penetrating therethrough in the Y direction. Here, the pulley groove 53b of the third guide pulley portion 53 has a second predetermined groove width D2 in the Y direction. The second predetermined groove width D2 is substantially equal to the first predetermined groove width D1. Further, the pulley groove 53b of the third guide pulley portion 53 is an example of a "groove of another pulley." The second predetermined groove width D2 is an example of a "groove width of another pulley."

### (Third Pulley Group)

As illustrated in FIG. 11, the surgical instrument 40 includes a third pulley group 5c that guides the first elongate element 1 engaged with the first pulley portion 51.

The third pulley group 5c is rotatably provided about a fourth shaft A4 at a position between the shaft 42 and the second shaft A2, and includes a fifth guide pulley portion 55 and a sixth guide pulley portion 56 that suspend the first elongate element 1. Here, the rotation axis R4 of the fourth shaft A4 is provided in parallel along the rotation axis R2 of the second shaft A2. The fifth guide pulley portion 55 and the sixth guide pulley portion 56 rotate about the rotation axis R4 of the fourth shaft A4. The fifth guide pulley portion 55 and the sixth guide pulley portion 56 are arranged at an end portion of the fourth shaft A4 on the Y1 side and an end portion of the fourth shaft A4 on the Y2 side, respectively. The fifth guide pulley portion 55 and the sixth guide pulley portion 56 are arranged at positions close to the shaft 42. The fifth guide pulley portion 55 and the sixth guide pulley portion 56 are examples of a "third pulley" and a "shaft-side pulley." The fourth shaft A4 is an example of a "pulley shaft." Further, the rotation axis R4 of the fourth shaft A4 is an example of a "rotation center axis of the shaft-side pulley."

Since the fifth guide pulley portion 55 and the sixth guide pulley portion 56 each have the same shape, only the fifth guide pulley portion 55 arranged at the end portion on the Y1 side will be described below.

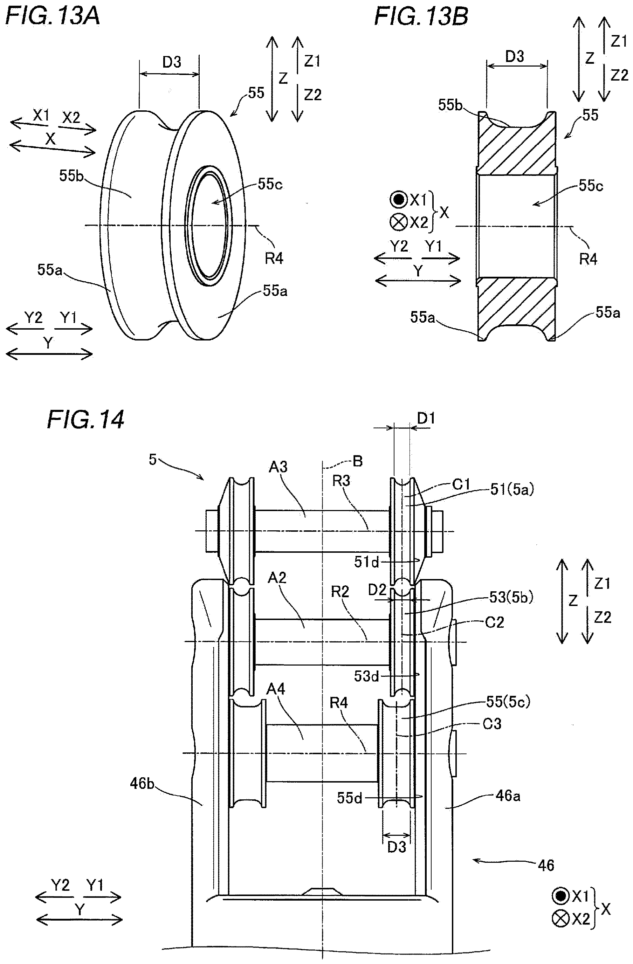

As illustrated in FIGS. 13A and 13B, the fifth guide pulley portion 55 is configured to rotate about the rotation axis R4 of the fourth shaft A4. Specifically, the fifth guide pulley portion 55 includes two disc portions 55a facing each other in the Y direction, a pulley groove 55b provided between the two disc portions 55a, and a shaft insertion hole 55c penetrating therethrough in the Y direction. Here, a width of the pulley groove 55b of the fifth guide pulley portion 55 has a third predetermined groove width D3 in the Y direction. Note that the third predetermined groove width D3 is an example of a "groove width of the shaft-side pulley." Further, the pulley groove 55b of the fifth guide pulley portion 55 is an example of a "groove of the shaft-side pulley."

### <Third Predetermined Groove Width>

The fifth guide pulley portion 55 and the sixth guide pulley portion 56 of the first embodiment have groove widths greater than those of the first guide pulley portion 51, the second guide pulley portion 52, the third guide pulley portion 53, and the fourth guide pulley portion 54.

Note that among the first guide pulley portion 51, the second guide pulley portion 52, the third guide pulley portion 53, the fourth guide pulley portion 54, the fifth guide pulley portion 55, and the sixth guide pulley portion 56, the first guide pulley portion 51, the third guide pulley portion 53, and the fifth guide pulley portion 55 will be described below.

As illustrated in FIG. 14, the third predetermined groove width D3 of the fifth guide pulley portion 55 which is closest to the shaft 42 among the first guide pulley portion 51, the third guide pulley portion 53, and the fifth guide pulley portion 55 (pulley group 5) is greater than the first predetermined groove width D1 of the first guide pulley portion 51 and the second predetermined groove width D2 of the third guide pulley portion 53. That is, the fifth guide pulley portion 55 and the sixth guide pulley portion 56 have grooves whose widths are greater than that of each of the first guide pulley portion 51, the second guide pulley portion 52, the third guide pulley portion 53, and the fourth guide pulley portion 54.

With this configuration, even if the wire W of the first elongate element 1 is obliquely suspended on the fifth guide pulley portion 55, the wire W of the first elongate element 1 is less likely to come into contact with a side portion (disk portion 55a) of the pulley groove 55b of the fifth guide pulley portion 55 on the first support body 45 side, so that the extending direction of the wire W of the first elongate element 1 is less likely to be changed. As a result, this can suppress an increase in the load applied to the wire W of the first elongate element 1 from the fifth guide pulley portion 55, so that the wire W of the first elongate element 1 can be smoothly guided.

The third predetermined groove width D3 is about 1.5 times the groove width of the greater one of the first predetermined groove width D1 and the second predetermined groove width D2. That is, the third predetermined groove width D3 has the size such that the guide pulley portion 55 can be arranged between the Y2 side end of the third protrusion 46a and the center B (schematically illustrated by the dash-dot-dash line) of the second support body 46 in the Y direction. Here, it is preferable that the third predetermined groove width D3 is 1.2 times or more and 2.0 times or less the first predetermined groove width D1 or the second predetermined groove width D2.

As a result, it is possible to secure the third predetermined groove width D3 of the fifth guide pulley portion 55, in which the wire W of the first elongate element 1 is less likely to come into contact with the side portions (the disk portions 55a) of the pulley groove 55b of the fifth guide pulley portion 55 even if the wire W of the first elongate element 1 is obliquely suspended on the fifth guide pulley portion 55. As a result, an increase in the load applied to the wire W of the first elongate element 1 from the fifth guide pulley portion 55 can be suppressed, so that the wire W of the first elongate element 1 can be more smoothly guided.

The center C3 (schematically illustrated by the dash-dot-dash line) in the Y direction of the pulley groove 55b of the fifth guide pulley portion 55 is arranged closer to the center B of the second support body 46 in the Y direction than the center C2 (schematically indicated by the dash-dot-dash line) in the Y direction of the pulley groove 53b of the third guide pulley portion 53 is. Further, the center C3 in the Y direction of the pulley groove 55b of the fifth guide pulley portion 55 is arranged closer to the center B of the second support body 46 in the Y direction than the center C1 (schematically indicated by the dash-dot-dash line) in the Y direction of the pulley groove 51b of the first guide pulley portion 51 is.

As a result, even if the wire W of the first elongate element 1 is obliquely suspended in the fifth guide pulley portion 55, the wire W of the first elongate element 1 is less likely to easily come into contact with the side portion (the disk portion 55a) of the pulley groove 55b of the fifth guide pulley portion 55 on the first support body 45 side. Further, the position of the wire W of the first elongate element 1 suspended on the fifth guide pulley portion 55 can be separated farther away from the second support body 46 than the position of the wire W of the first elongate element 1 suspended on the third guide pulley portion 53 is. Therefore, it is possible to suppress the contact between the wire W of the first elongate element 1 and the second support body 46.

The center C3 in the Y direction of the pulley groove 55b of the fifth guide pulley portion 55 is arranged at a position farther away in the Y direction than the center B of the second support body 46 in the Y direction.

The position of an end portion 55d of the fifth guide pulley portion 55 on the side opposite to the center B of the second support body 46 in the Y direction and the position of an end portion 53d of the third guide pulley portion 53 on the side opposite to the center B of the second support body 46 in the Y direction are substantially the same as each other, when viewed in the X1 direction. Further, the position of the end portion 55d of the fifth guide pulley portion 55 on the side opposite to the center B of the second support body 46 in the Y direction and the position of an end portion 51d of the first guide pulley portion 51 on the side opposite to the center B of the second support body 46 in the Y direction are substantially the same as each other, when viewed in the X1 direction.

Accordingly, it is possible to prevent the end portion 55d of the fifth guide pulley portion 55 from protruding toward the Y1 direction than the end portion 51d of the first guide pulley portion 51 and the end portion 53d of the third guide pulley portion 53. As a result, it is possible to suppress an increase in the length of the second shaft A2 that supports the fifth guide pulley portion 55, so as to suppress an increase in the size of the second support body 46. Further, it is possible to prevent the arrangement positions of the first guide pulley portion 51, the third guide pulley portion 53, and the fifth guide pulley portion 55 from being complicated. As a result, it is possible to further suppress the increase the load applied to the wire W of the first elongate element 1 due to a complicated path of the elongate element 44 passing through the first guide pulley portion 51, the third guide pulley portion 53, and the fifth guide pulley portion 55.

That is, the end portion 55d of the fifth guide pulley portion 55 on the side opposite to the center B of the second support body 46 in the Y direction and the end portion 53d of the third guide pulley portion 53 on the side opposite to the center B of the second support body 46 in the Y direction are arranged on the same plane (XZ plane) when viewed in the X1 direction. Further, the end portion 55d of the fifth guide pulley portion 55 on the side opposite to the center B in the Y direction of the second support body 46 and the end portion 51d of the guide pulley portion 51 on the side opposite to the center B the second support body 46 in the Y direction are arranged on the same plane (XZ plane).

The extending direction of the rotation axis R4 of the fifth guide pulley portion 55 and the extending direction of the rotation axis R2 of the third guide pulley portion 53 are substantially parallel when viewed in the X1 direction. Further, the extending direction of the rotation axis R4 of the fifth guide pulley portion 55 and the extending direction of the rotation axis R3 of the first guide pulley portion 51 are substantially parallel when viewed in the X1 direction.

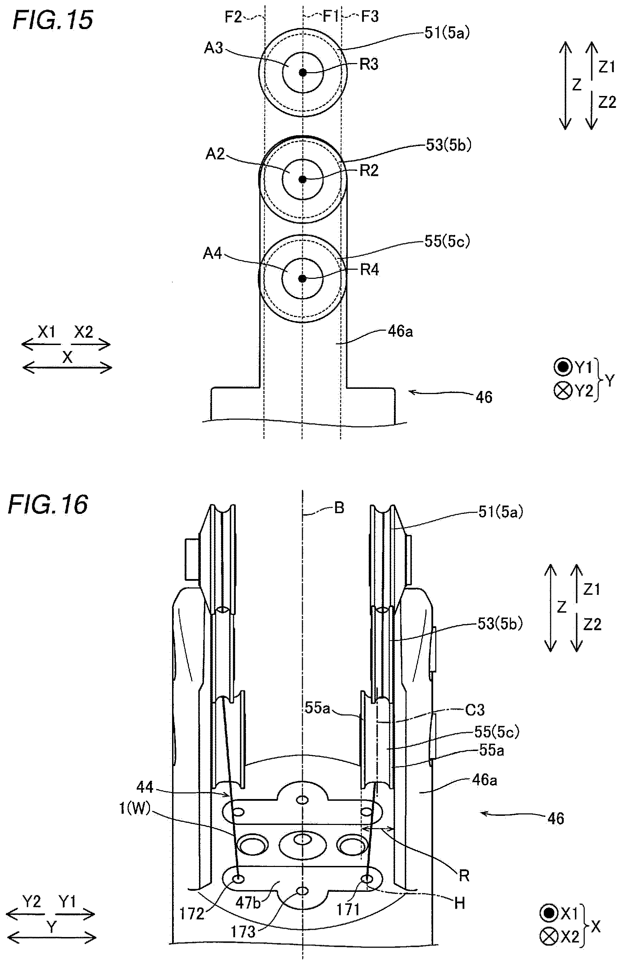

As illustrated in FIG. 15, the position of the rotation axis R4 of the fifth guide pulley portion 55 and the position of the rotation axis R2 of the third guide pulley portion 53 are arranged on a straight line F1 along the Z direction (the direction in which the shaft 42 extends) when viewed from the Y1 direction. Further, the position of the rotation axis R4 of the fifth guide pulley portion 55 and the position of the rotation axis R3 of the first guide pulley portion 51 are arranged on the straight line F1 along the Z direction (the direction in which the shaft 42 extends) when viewed from the Y1 direction.

With this, it is possible to suppress an increase in the tension of the wire W of the first elongate element 1 suspended on the first guide pulley portion 51, the third guide pulley portion 53, and the fifth guide pulley portion 55, compared with a case where the arrangement positions of the first guide pulley portion 51, the third guide pulley portion 53, and the fifth guide pulley portion 55 are shifted from the straight line F1 along the Z direction when viewed from the Y1 direction. As a result, it is possible to further suppress an increase in the load applied to the wire W of the first elongate element 1 and the first guide pulley portion 51.

Further, the most X1 side position of the pulley groove 51b of the first guide pulley portion 51, the most X1 side position of the pulley groove 53b of the third guide pulley portion 53, and the most X1 side position of the pulley groove 55b of the fifth guide pulley portion 55 are arranged on a straight line F2 when viewed from the Y1 direction. The most X2 side position of the pulley groove 51b of the first guide pulley portion 51, the most X2 side position of the pulley groove 53b of the third guide pulley portion 53 , and the most X2 side position of the pulley groove 55b of the fifth guide pulley portion 55 are arranged on a straight line F3 when viewed from the Y1 direction.

Further, the first guide pulley portion 51, the third guide pulley portion 53, and the fifth guide pulley portion 55 are formed to have substantially the same shape when viewed from the Y1 direction.

As illustrated in FIG. 16, when viewed in the X1 direction, the first insertion hole 171 of the second seal portion 47b of the silicon seal 47 is arranged between both end portions of the fifth guide pulley portion 55 in the Y direction. That is, when viewed in the X1 direction, the first insertion hole 171 of the second seal portion 47b of the silicon seal 47 is arranged within a space R between the two disc portions 55a of the fifth guide pulley portion 55.

With this, the wire W of the first elongate element 1 is less likely to come into contact with the side portion (disk portion 55a) of the pulley groove 55b of the fifth guide pulley portion 55 on the first support 45 side, and thus the extending direction of the wire W can be less likely to be changed. As a result, an increase in the load applied to the wire W of the first elongate element 1 from the fifth guide pulley portion 55 can be suppressed, so that the wire W of the first elongate element 1 can be more smoothly guided.

Here, when viewed in the X1 direction, the position of the Y2 side end portion of the first insertion hole 171 of the second seal portion 47b of the silicon seal 47 is substantially the same as the position of the Y2 side end portion of the fifth guide pulley portion 55.

Further, when viewed in the X1 direction, a center C3 in the Y direction of the fifth guide pulley portion 55 is arranged farther away from the center B in the Y direction of the second support body 46 than the center H in the Y direction of the first insertion hole 171 of the second seal portion 47b of the silicon seal 47 is. That is, the center C3 (schematically illustrated by the dash-dot-dash line) in the Y direction of the fifth guide pulley portion 55 is arranged on the Y1 side with respect to the center H in the Y direction of the first insertion hole 171 of the second seal portion 47b of the silicon seal 47.

Note that the second guide pulley portion 52, the fourth guide pulley portion 54, and the sixth guide pulley portion 56 have the same configurations as the first guide pulley portion 51, the third guide pulley portion 53, and the fifth guide pulley portion 55, respectively.

### (First Modification)

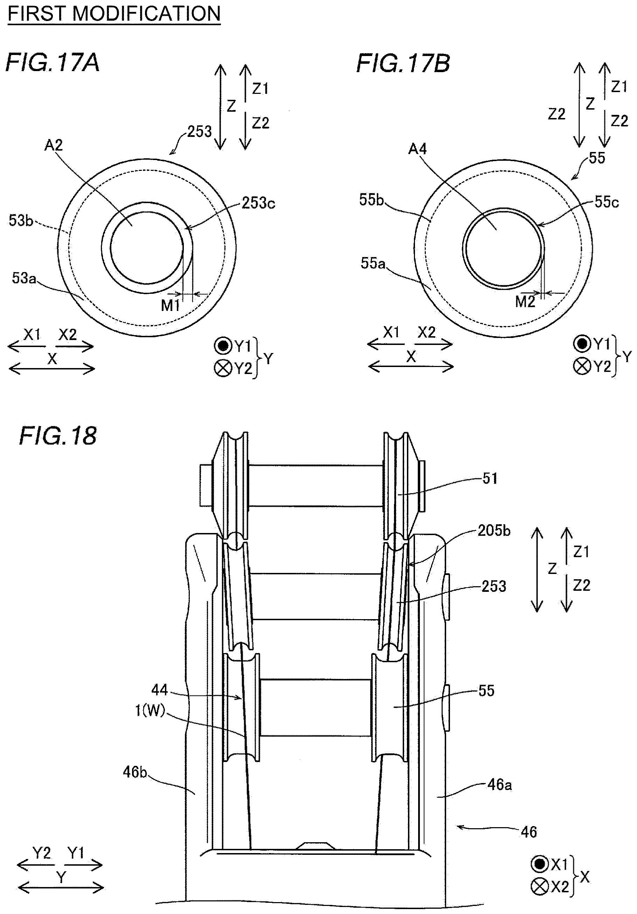

Next, a first modification is described with reference to FIGS. 17 and 18. In this first modification, an example is described in which the configuration of a second pulley group 205b is different from that in the first embodiment. Note that in the drawings, the constituents same as in the embodiment described above are designated by the same reference numerals.

Hereinafter, a third guide pulley portion 253 and a fifth guide pulley portion 55 are described. Note that the third guide pulley portion 253 is an example of "another pulley."

The third guide pulley portion 253 is configured to rattle with respect to the second shaft A2. Specifically, as illustrated in FIG. 17A, the third guide pulley portion 253 has a gap M1 between a shaft insertion hole 253c of the third guide pulley portion 253 and the second shaft A2. That is, the third guide pulley portion 253 is provided so as to be swingable within a predetermined range. Here, as illustrated in FIG. 17B, the fifth guide pulley portion 55 has a gap M2 between the shaft insertion hole 55c of the fifth guide pulley portion 55 and the fourth shaft A4. Note that the gap M2 is a value close to about 0. Note that the shaft insertion hole 253c of the third guide pulley portion 253 is an example of "another shaft insertion hole."

The gap M1 between the second shaft A2 and the shaft insertion hole 253c of the third guide pulley portion 253 which is arranged adjacent to the fifth guide pulley portion 55 is greater than the gap M2 between the fourth shaft A4 and the shaft insertion hole 55c of the fifth guide pulley portion 55. That is, as illustrated in FIG. 18, the fifth guide pulley portion 55 is suppressed from being tilted with respect to the fourth shaft A4. On the other hand, the third guide pulley portion 253 is allowed to be tilted with respect to the second shaft A2 along the extending direction of the wire W of the first elongate element 1 being suspended thereon. Note that not only the third guide pulley portion 253 but also the fourth guide pulley portion 254 on the Y2 side have the above described configuration. Further, the other configurations in the first modification are the same as those in the first embodiment.

As a result, the third guide pulley portion 253 can be tilted along the extending direction of the wire W of the first elongate element 1, so that an increase in the load applied to the wire W of the first elongate element 1 can be further suppressed.

### [Second Embodiment]

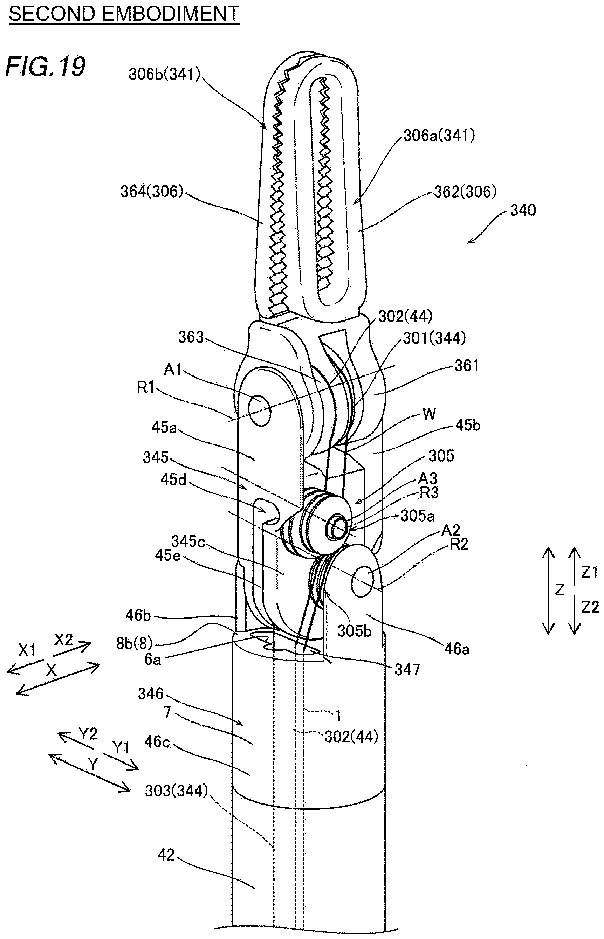

Next, a second embodiment of the invention is explained with reference to FIGS. 19 to 29. In the second embodiment, unlike the above-described first embodiment in which the number of the end effector member 5 of the end effector 41 is one, an example of the configuration in which the number of end effector members 205 of an end effector 341 is two is described. In the drawings, the constituents same as in a first embodiment are designated by the same reference numerals.

### (Surgical Instrument)

As illustrated in FIG. 19, a surgical instrument 340 includes an elongate element 344, an end effector 341, a first support body 345, a second support body 346, a silicone seal 347, a pulley group 305, and a shaft 42. The pulley group 305 includes a first pulley group 305a and a second pulley group 305b.

The first support body 345 rotatably supports the end effector 41 about a first shaft A1. The second support body 346 rotatably supports the first support body 345 about a second shaft A2. Note that the rotation axis R2 of the second shaft A2 is an example of a "rotation center axis of a shaft-side pulley." The second shaft A2 is an example of a "pulley shaft of a shaft-side pulley."

The end effector 341 is configured to perform procedures on the surgery site of the patient P based on the function of a type of the end effector 341. Specifically, the end effector 341 includes plural (two) end effector members 306. In other words, the end effector members 306 are a first jaw 306a and a second jaw 306b. The first jaw 306a and the second jaw 306b are attached to the first support body 345.

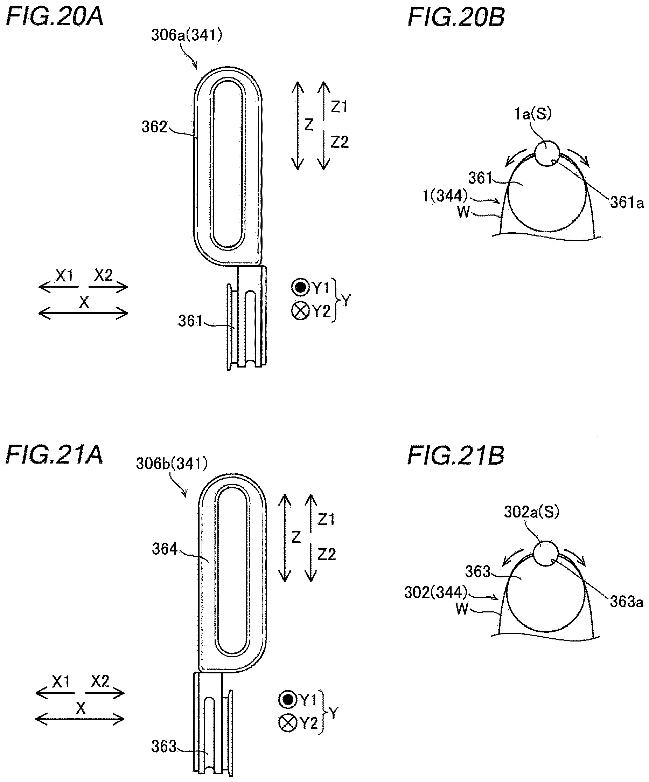

As illustrated in FIGS. 20A and 20B, the first jaw 306a includes a first pulley portion 361 and a first procedure portion 362. The first pulley portion 361 is formed with a first recess 361a engaged with an attachment S of the elongate element 344 (hereinafter, an attachment 1a of a first elongate element 1). The procedure portion 362 changes the orientation by rotation of the first pulley portion 361 in accordance with movement of the first elongate element 1. The attachment 1a of the first elongate element 1 is formed in a circular column shape. As illustrated in FIGS. 21A and 21B, the second jaw 306b includes a second pulley portion 363 and a second procedure portion 364. The second pulley portion 363 is formed with a second recess 363a engaged with an attachment S of the elongate element 344 (hereinafter, an attachment 302a of a second elongate element 302). The procedure portion 364 changes the orientation by rotation of the second pulley portion 363 in accordance with movement of the second elongate element 302. The attachment 302a of the second elongate element 302 is formed in a circular column shape.

### (First Support Body)

As illustrated in FIG. 19, the first support body 345 includes a first protrusion portion 45a, a second protrusion portion 45b, a third pulley portion 345c, and a through hole 45d. The first protrusion portion 45a protrudes in the Z1 direction from an end portion on the X1 side of the third pulley portion 345c. The first protrusion portion 45a supports an end portion on the X1 side of the first shaft A1. The second protrusion portion 45b protrudes in the Z1 direction from an end portion on the X2 side of the third pulley portion 345c. The second protrusion portion 45b supports an end portion on the X2 side of the first shaft A1. The third pulley portion 345c is rotatably supported by the second shaft A2. The third pulley portion 345c includes a pulley groove 45e formed along a circumferential direction of the second shaft A2. To the third pulley portion 345c, an elongate element 44 (hereinafter referred to as a third elongate element 303) is suspended, in order to rotate the first support body 45. The through hole 45d has a size through which the third elongate element 303 can be inserted. The through hole 45d is configured to linearly pass through the first support body 345 along the X direction.

### (Second Support Body)

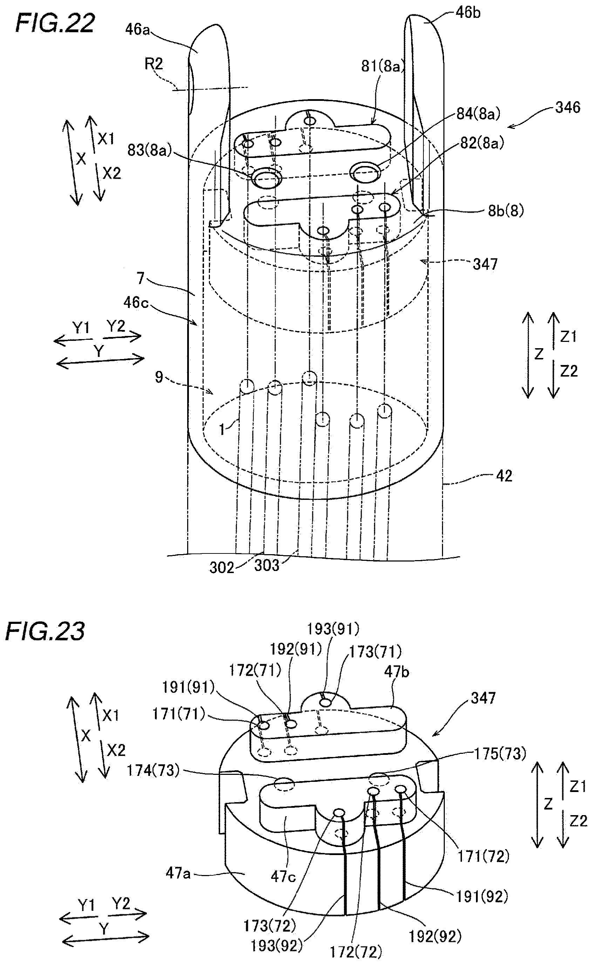

As illustrated in FIG. 22, the second support body 346 includes a third protrusion portion 46a, a fourth protrusion portion 46b, and a connection base portion 46c. The third protrusion portion 46a protrudes in the Z1 direction from a Y1 side end portion of the connection base portion 46c. The third protrusion portion 46a supports a Y1 side end portion of the second shaft A2. The third protrusion portion 46a supports a Y1 side end portion of the second shaft A2. The fourth protrusion portion 46b protrudes in the Z1 direction from a Y2 side end portion of the connection base portion 46c. The fourth protrusion portion 46b supports a Y2 side end portion of the second shaft A2. A Z2 side end portion of the connection base portion 46c is connected to a Z1 side end portion of the shaft 42.

The connection base portion 46c is formed in a substantially cylindrical tubular shape. The connection base portion 46c includes a side surface portion 7 and an end surface portion 8. The side surface portion 7 is provided along a circumferential direction around an axis extending in the Z direction. The end surface portion 8 is provided in an end portion of the side surface portion 7 on the other side opposite to the shaft 42 (an end portion on the Z1 side). The connection base portion 46c includes an inner space 9 surrounded by the side surface portion 7 and the end surface portion 8. The inner space 9 of the connection base portion 46c is opened toward the Z2 direction.

The end surface portion 8 includes a partition wall 8b including communication holes 8a passing through the partition wall 8b in the extending direction of the shaft 42 (the Z direction). The partition wall 8b is configured to separate the inner space 9 and the outer space of the connection base portion 46c.

The partition wall 8b is formed with the communication holes 8a passing through the partition wall 8b in the extending direction of the shaft 42 (the Z direction). Specifically, the second support body 346 includes the communication holes 8a that penetrate, in the extending direction of the shaft 42 (the Z direction), the end surface portion 8 on the opposite side of the shaft 42 . The communication holes 8a allow communication between the inner space 9 of the connection base portion 46c and the outer space of the connection base portion 46c. In the partition wall 8b, a communication hole 8a located on the X1 side (hereinafter, a first communication hole 81) and a communication hole 8a located on the X2 side (hereinafter, a second communication hole 82) are formed. Each of the first communication hole 81 and the second communication hole 82 is formed in a substantial T-shape as seen from the Z1 direction.

In the partition wall 8b, a communication hole 8a located on the Y1 side (hereinafter, a third communication hole 83) and a communication hole 8a located on the Y2 side (hereinafter, a fourth communication hole 84) are also formed. Each of the third communication hole 83 and the fourth communication hole 84 is formed in a substantially circular shape as seen from the Z1 direction.

### (Silicone Seal)

As illustrated in FIGS. 22 and 23, a silicone seal 347 is inserted in the inner space 9 of the second support body 346. The silicone seal 347 is arranged in an end portion of the second support body 346 on the Z1 side of the inner space 9. The silicone seal 347 is configured to seal (close) the first communication hole 81 and the second communication hole 82. The silicone seal 347 is an example of a "seal member."

Here, the silicone seal 347 includes a first seal portion 47a, a second seal portion 47b, and a third seal portion 47c. The first seal portion 47a is formed in an H-shape as seen from the Z1 direction. The first seal portion 47a is in close contact with the surface of the end surface portion 8 of the second support body 346 on the X2 side. On the first seal portion 47a, the second seal portion 47b is arranged on the X1 side, and the third seal portion 47c is arranged on the X2 side.

The second seal portion 47b is formed correspondingly to the first communication hole 81 of the second support body 346. Specifically, the second seal portion 47b is formed in a substantial T-shape as seen from the Z1 direction correspondingly to the shape of the first communication hole 81 as seen from the Z1 direction. The third seal portion 47c is formed correspondingly to the second communication hole 82 of the second support body 346. Specifically, the third seal portion 47c is formed in a substantial T-shape as seen from the Z1 direction correspondingly to the shape of the second communication hole 82 as seen from the Z1 direction.

The silicone seal 347 includes a first insertion hole group 71, a second insertion hole group 72, and a third insertion hole group 73.

The first insertion hole group 71 and the second insertion hole group 72 each include a first insertion hole 171 through which the wire W of the first elongate element 1 is inserted, a second insertion hole 172 through which the wire W of the second elongate element 302 is inserted, and a third insertion hole 173 through which the wire W of the third elongate element 303 is inserted. The third insertion hole group 73 includes a fourth insertion hole 174 and a fifth insertion hole 175 through which electric wires are inserted that supply power to the end effector 341. The fourth insertion hole 174, the fifth insertion hole 175 of the third insertion hole group 73 pass through only the first seal portion 47a in the Z direction.

The silicone seal 347 includes a first slit group 91 and a second slit group 92. The first slit group 91 includes a first slit 191 that is connected to the first insertion hole 171 of the first insertion hole group 71 and guides the wire W of the first elongate element 1 to the first insertion hole 171 of the first insertion hole group 71. The first slit group 91 includes a second slit 192 that is connected to the second insertion hole 172 of the first insertion hole group 71 and guides the wire W of the second elongate element 302 to the second insertion hole 172 of the first insertion hole group 71. The first slit group 91 includes a third slit 193 that is connected to the third insertion hole 173 of the first insertion hole group 71 and guides the wire W of the third elongate element 303 to the third insertion hole 173 of the first insertion hole group 71. Similarly, the second slit group 92 also includes a first slit 191, a second slit 192, and a third slit 193 so as to correspond to the first insertion hole 171 and the second insertion hole 172 and the third insertion hole 173 of the second insertion hole group 72, respectively.

### (First Pulley Group)

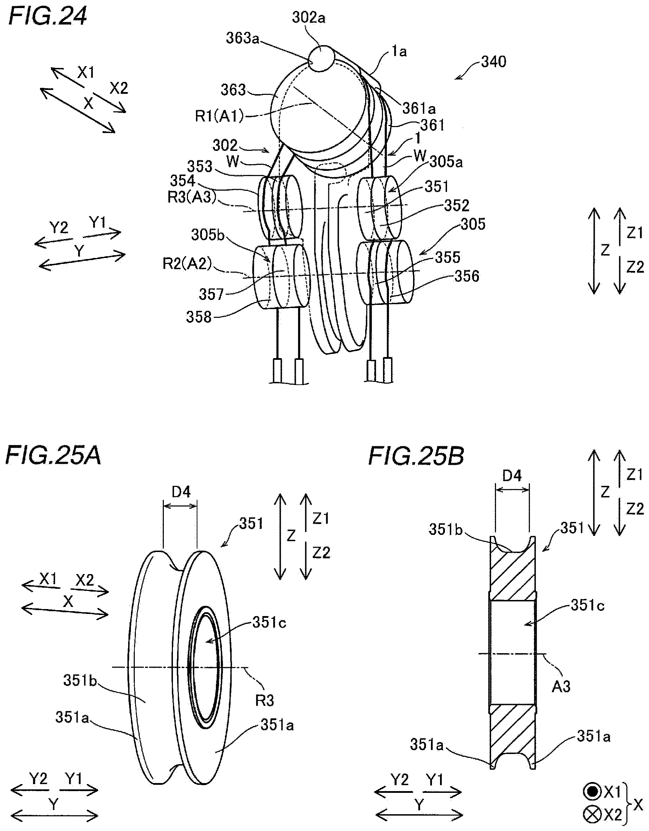

As illustrated in FIG. 24, the surgical instrument 340 includes a first pulley group 305a that guides the first elongate element 1 engaged with the first pulley portion 361 and the second elongate element 302 engaged with the second pulley portion 363.

The first pulley group 305a includes a first inner pulley portion 351 and a first outer pulley portion 352, and a second inner pulley portion 353 and a second outer pulley portion 354, which are rotatably provided about the third shaft A3 at the position between the first shaft A1 and the second shaft A2. The first inner pulley portion 351 and the first outer pulley portion 352 and the second inner pulley portion 353 and the second outer pulley portion 354 suspend the first elongate element 1 and the second elongate element 302. Note that the first inner pulley portion 351, the first outer pulley portion 352, the second inner pulley portion 353, and the second outer pulley portion 354 are examples of a "first pulley" and "another pulley." The third shaft A3 is an example of "another pulley shaft." The rotation axis R3 of the third shaft A3 is an example of a "rotation center axis of another pulley."

Here, the first inner pulley portion 351 and the second inner pulley portion 353 rotate about the rotation axis R3 of the third shaft A3, respectively. The first outer pulley portion 352 and the second outer pulley portion 354 rotate about the rotation axis R3 of the third shaft A3, respectively. The first outer pulley portion 352 and the second outer pulley portion 354 are arranged at the end portion of the third shaft A3 on the Y1 side and the end portion of the first shaft A1 on the Y2 side, respectively. The first inner pulley portion 351 and the second inner pulley portion 353 are arranged on the Y2 side of the first outer pulley portion 352 and the Y1 side of the second outer pulley portion 354, respectively.

Since the first inner pulley portion 351 and the first outer pulley portion 352, the second inner pulley portion 353, and the second outer pulley portion 354 have the same shape as each other, only the first inner pulley portion 351 is described below.

As illustrated in FIGS. 25A and 25B, the first inner pulley portion 351 is configured to rotate about the rotation axis R3 of the third shaft A3. Specifically, the first inner pulley portion 351 includes two disk portions 351a facing each other in the Y direction, a pulley groove 351b provided between the two disk portions 351a, and a shaft insertion hole 351c penetrating therethrough in the Y direction. Here, the width of the pulley groove 351b of the first inner pulley portion 351 has a fourth predetermined groove width D4 in the Y direction. The fourth predetermined groove width D4 is an example of a "groove width of another pulley." Further, the pulley groove 351b of the first inner pulley portion 351 is an example of a "groove of another pulley."

### (Second Pulley Group)

As illustrated in FIG. 24, the surgical instrument 340 includes a second pulley group 305b that guides the first elongate element 1 engaged with the first pulley portion 361. The second pulley group 305b guides the second elongate element 302 engaged with the second pulley portion 363.

The second pulley group 305b includes a third inner pulley portion 355 and a third outer pulley portion 356, and a fourth inner pulley portion 357 and a fourth outer pulley portion 358, which are rotatably provided about the second shaft A2. That is, the third inner pulley portion 355, the third outer pulley portion 356, the fourth inner pulley portion 357, and the fourth outer pulley portion 358 are arranged on the second shaft A2. Note that the third inner pulley portion 355, the third outer pulley portion 356, the fourth inner pulley portion 357, and the fourth outer pulley portion 358 are examples of a "second pulley" and a "shaft-side pulley."

The third inner pulley portion 355, the third outer pulley portion 356, the fourth inner pulley portion 357, and the fourth outer pulley portion 358 suspend thereon the first elongate element 1 and the second elongate element 302. The third inner pulley portion 355, the third outer pulley portion 356, the fourth inner pulley portion 357, and the fourth outer pulley portion 358 rotate about the rotation axis R2 of the second shaft A2, respectively. The third outer pulley portion 356 and the fourth outer pulley portion 358 are arranged at the end portion of the second shaft A2 on the Y1 side and the end portion of the second shaft A2 on the Y2 side, respectively. The third inner pulley portion 355 and the fourth inner pulley portion 357 are arranged on the Y2 side of the third outer pulley portion 356 and the Y1 side of the fourth outer pulley portion 358, respectively. The third inner pulley portion 355, the third outer pulley portion 356, the fourth inner pulley portion 357, and the fourth outer pulley portion 358 are arranged at positions close to the shaft 42.

Since the third inner pulley portion 355, the third outer pulley portion 356, the fourth inner pulley portion 357, and the fourth outer pulley portion 358 each have the same shape, only the third inner pulley portion 355 is described below.

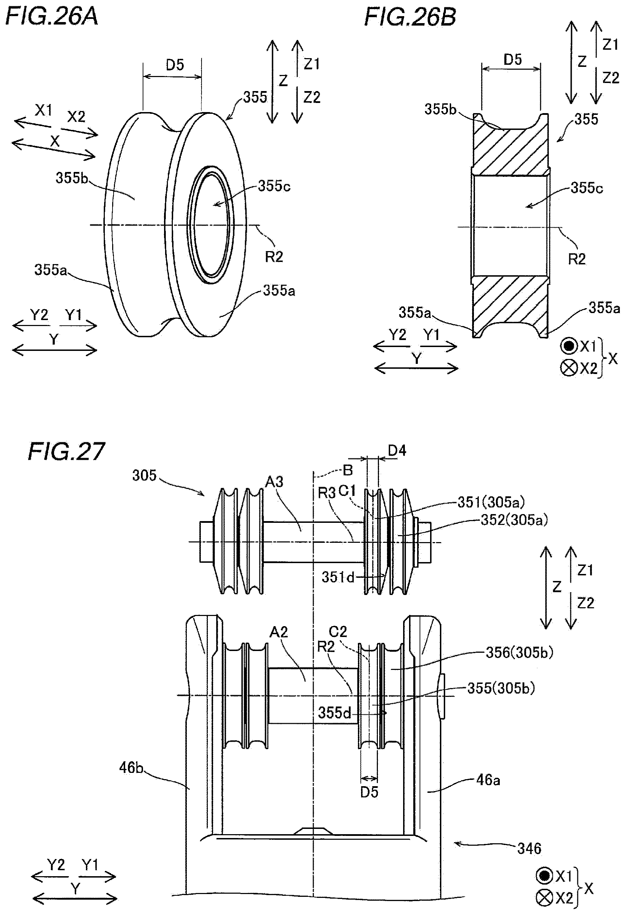

As illustrated in FIGS. 26A and 26B, the third inner pulley portion 355 is configured to rotate about the rotation axis R2 of the second shaft A2. Specifically, the third inner pulley portion 355 includes two disk portions 355a facing each other in the Y direction, a pulley groove 355b provided between the two disk portions 355a, and a shaft insertion hole 355c penetrating therethrough in the Y direction. Here, the width of the pulley groove 355b of the third inner pulley portion 355 has a fifth predetermined groove width D5 in the Y direction. Note that the fifth predetermined groove width D5 is an example of a "groove width of a shaft-side pulley." Further, the pulley groove 355b of the third inner pulley portion 355 is an example of a "groove of a shaft-side pulley."

### <Fifth Predetermined Groove Width>

As illustrated in FIG. 27, the third inner pulley portion 355, the third outer pulley portion 356, the fourth inner pulley portion 357, and the fourth outer pulley portion 358 according to the second embodiment have groove widths greater than those of the inner pulley portion 351, the first outer pulley portion 352, the second inner pulley portion 353, and the second outer pulley portion 354.

As a result, the wire W of the first elongate element 1 and the wire W of the second elongate element 2 are less likely to come into contact with the side portion (disk portion 355a) of the third inner pulley portion 355, the third outer pulley portion 356, the fourth inner pulley portion 357, and the fourth outer pulley portion 358. Therefore, the extending directions of the wire W of the first elongate element 1 and the wire W of the second elongate element 302 are less likely to be changed. As a result, the increase in the load applied from the wire W of the first elongate element 1 and the wire W of the second elongate element 2 can be suppressed by the third inner pulley portion 355, the third outer pulley portion 356, the fourth inner pulley portion 357, and the fourth outer pulley portion 358, and thus the wire W of the first elongate element 1 and the wire W of the second elongate element 302 can be guided more smoothly.

Note that in the following, only the first inner pulley portion 351 among the first inner pulley portion 351 and the first outer pulley portion 352, the second inner pulley portion 353, and the second outer pulley portion 354 and only the third inner pulley portion 355 among the third inner pulley portion 355, the third outer pulley portion 356, the fourth inner pulley portion 357, and the fourth outer pulley portion 358 are described below.

As illustrated in FIG. 27, the fifth predetermined groove width D5 of the third inner pulley portion 355, which is provided at a position closest to the shaft 42 among the first inner pulley portion 351 and the third inner pulley portion 355, is greater than the fourth predetermined groove width D4 of the first inner pulley portion 351.

The fifth predetermined groove width D5 is about 1.5 times the groove width of the fourth predetermined groove width D4. That is, the fifth predetermined groove width D5 has the size such that the third inner pulley portion 355 can be arranged between the Y2 side end portion of the third protrusion 46a and the center B (schematically indicated by the dash-dot-dash line) of the second support body 346 in the Y direction. Here, it is preferable that the fifth predetermined groove width D5 is 1.2 times or more and 2.0 times or less of the fourth predetermined groove width D4.

The center C2 (schematically illustrated by the dash-dot-dash line) in the Y direction of the pulley groove 355b of the third inner pulley portion 355 is arranged closer to the center B in the Y direction of the second support body 346 than the center C1 (schematically illustrated by the dash-dot-dash line) in the Y direction of the pulley groove 351b of the first inner pulley portion 351.

The center C2 in the Y direction of the pulley groove 355b of the third inner pulley portion 355 is arranged farther away in the Y direction than the center B in the Y direction of the second support body 346.

As illustrated in FIG. 27, when viewed in the X1 direction, the position of an end portion 355d of the third inner pulley portion 355 on the side opposite to the center B in the Y direction of the second support body 346 and the position of an end portion 351d of the first inner pulley portion 351 on the side opposite to the center in the Y direction of the second support body 346 are substantially the same as each other. That is, the end portion 355d of the third inner pulley portion 355 on the side opposite to the center B of the second support body 346 in the Y direction and the end portion 351d of the inner pulley portion 351 on the side opposite to the center B of the second support body 346 in the Y direction are arranged on the same plane (XZ plane), when viewed in the X1 direction.

The extending direction of the rotation axis R2 of the third inner pulley portion 355 and the extending direction of the rotation axis R3 of the first inner pulley portion 351 are substantially parallel when viewed in the X1 direction.

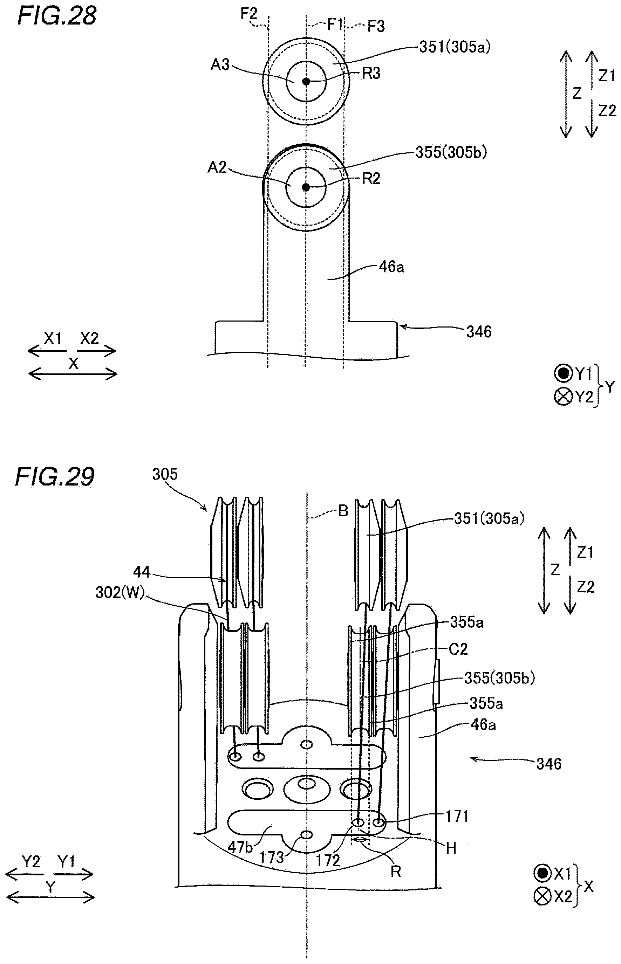

As illustrated in FIG. 28, the position of the rotation axis R2 of the third inner pulley portion 355 and the position of the rotation axis R3 of the first inner pulley portion 351 are arranged on the straight line F1 along the Z direction (the direction in which the shaft 42 extends), when viewed from the Y1 direction.

Further, the most X1 side position of the pulley groove 351b of the first inner pulley portion 351 and the most X1 side position of the pulley groove 355b of the third inner pulley portion 355 are arranged on the straight line F2 when viewed from the Y1 direction. The most X2 side position of the pulley groove 351b of the first inner pulley portion 351 and the most X2 side position of the pulley groove 355b of the third inner pulley portion 355 are arranged on the straight line F3 when viewed from the Y1 direction.

Further, the first inner pulley portion 351 and the third inner pulley portion 355 are formed to have substantially the same shape when viewed from the Y1 direction.

As illustrated in FIG. 29, when viewed in the X1 direction, the second insertion hole 172 of the second seal portion 47b of the silicon seal 347 is arranged between both ends of the third inner pulley portion 355 in the Y direction. That is, when viewed in the X1 direction, the second insertion hole 172 of the second seal portion 47b of the silicon seal 347 is arranged within the space R between the pair of the disk portions 355a of the third inner pulley portion 355.

Here, when viewed in the X1 direction, the position of the Y2 side end portion of the second insertion hole 172 of the second seal portion 47b of the silicon seal 347 and the position of the Y2 side end portion of the third inner pulley portion 355 are substantially the same.

Further, when viewed in the X1 direction, the center C2 in the Y direction of the third inner pulley portion 355 is arranged farther away from the center B of the second support body 346 in the Y direction than the center H in the Y direction of the second insertion hole 172 of the second seal portion 47b of the silicon seal 347. That is, the center C2 in the Y direction of the third inner pulley portion 355 is arranged on the Y1 side with respect to the center H in the Y direction of the second insertion hole 172 of the second seal portion 47b of the silicon seal 347.

Note that the first outer pulley portion 352, the second inner pulley portion 353, the second outer pulley portion 354, the third outer pulley portion 356, the fourth inner pulley portion 357, and the fourth outer pulley portion 358 also have the same configuration described above.

In this way, even when the number of the end effector members 306 is two, it is possible to suppress an increase in the load applied to the wire W of the first elongate element 1 and the wire W of the second elongate element 302 by means of the third inner pulley portion 355, the third outer pulley portion 356, the fourth inner pulley portion 357, and the fourth outer pulley portion 358.

### (Second Modification)

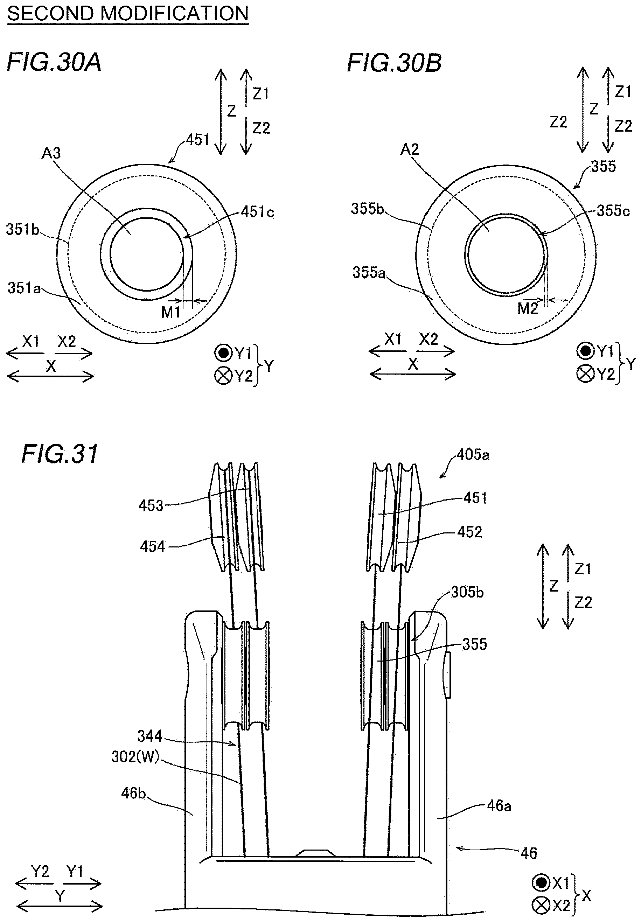

Next, a second modification is described with reference to FIGS. 30 and 31. In this second modification, an example in which the configuration of a first pulley group 405a is different from that of the second embodiment is described. Note that in the drawings, the constituents same as in the embodiment described above are designated by the same reference numerals.

Hereinafter, a first inner pulley portion 451 and a third inner pulley portion 355 are described. Note that the first inner pulley portion 451 is an example of "another pulley."

The first inner pulley portion 451 is configured to rattle with respect to the third shaft A3. Specifically, as illustrated in FIG. 30A, the first inner pulley portion 451 has a gap M1 between a shaft insertion hole 451c of the first inner pulley portion 451 and the third shaft A3. That is, the first inner pulley portion 451 is provided so as to be swingable within a predetermined range. Here, as illustrated in FIG. 30B, the third inner pulley portion 355 has a gap M2 between the shaft insertion hole 355c of the third inner pulley portion 355 and the second shaft A2. Note that the gap M2 is a value close to about 0. The shaft insertion hole 451c of the first inner pulley portion 451 is an example of "another shaft insertion hole."

The gap M1 between the third shaft A3 and the shaft insertion hole 451c of the first inner pulley portion 451 arranged adjacent to the third inner pulley portion 355 is greater than the gap M2 between the second shaft A2 and the shaft insertion hole 355c of the third inner pulley portion 355. That is, as illustrated in FIG. 31, the third inner pulley portion 355 is suppressed from being titled with respect to the second shaft A2. On the other hand, the first inner pulley portion 451 is allowed to be tilted with respect to the second shaft A2 along the extending direction of the wire W of the first elongate element 1 suspended thereon. Note that the first outer pulley portion 452, the second inner pulley portion 453, and the second outer pulley portion 454 also have the same configuration described above. Further, the other configurations in the second modification are the same as those in the embodiment described above.

### (Modifications)

It should be understood that one or more embodiments described above are illustrated by way of example in every respect and not limit the disclosure. The scope of the invention is indicated by claims, not by explanation of the one or more embodiments described above, and includes equivalents to the claims and all alterations (modifications) within the same.

For example, although the example has been described in the second embodiment in which the third inner pulley portion 355, the third outer pulley portion 356, the fourth inner pulley portion 357, and the fourth outer pulley portion 358 have the fifth predetermined groove width D5 wider than the first inner pulley portion 351, the first outer pulley portion 352, the second inner pulley portion 353, and the second outer pulley portion 354, the invention is not limited to this. In the invention, only the third inner pulley portion and the fourth inner pulley portion may be arranged at the position on the center side in the Y direction of the first support body and have the fifth predetermined groove widths wider than those of the first inner pulley portion, the first outer pulley portion, the second inner pulley portion, and the second outer pulley portion. As a result, since the groove widths of the third inner pulley portion and the fourth inner pulley portion arranged at the position on the center side where the extending direction of the elongate element is likely to be changed are set wider than the groove widths of at least the first inner pulley portion and the second inner pulley portion, it is possible to effectively suppress an increase in the load applied to the elongate element from the third inner pulley portion and the fourth inner pulley portion.

Further, in the first and second embodiments, the surgical instrument has the first jaw and the second jaw as the end effector member or the hook as the end effector member, but the invention is not limited to this. In the invention, the surgical instrument may use scissors, a high-frequency knife, a snare wire, a clamp, a stapler, or the like as an end effector member.

### EXPLANATION OF REFERENCE NUMERALS

5, 305: Pulley group (a plurally of pulleys),
6, 306: End effector member,
8: End surface portion,
8a: Communication hole,
40, 340: Surgical instrument (robotic surgical instrument),
41, 341: End effector,
42 : Shaft,
44, 344: Elongate element,
45, 345: First support body,
46, 346: Second support body,
47, 347: Silicon seal (seal member),
51: First guide pulley (first pulley, another pulley),
51c, 53c, 55c, 351c, 355c: Shaft insertion hole,
51d: End portion of first guide pulley portion (end portion on the opposite side),
52: Second guide pulley portion (first pulley, another pulley),
53: Third guide pulley portion (second pulley, another pulley),
53d: End portion of third guide pulley portion (end portion on the opposite side), 54: Fourth guide pulley portion (second pulley, another pulley),
55: Fifth guide pulley portion (shaft-side pulley, third pulley),
55a, 355a: Disc portion (both end portions),
55d: End portion of third guide pulley portion (end portion on the opposite side),
56: Sixth guide pulley portion (shaft- side pulley, third pulley),
171: First insertion hole (insertion hole),
172: Second insertion hole (insertion hole),
351: First inner pulley portion (first pulley, another pulley),
351d: End portion of first inner pulley portion (end portion on the opposite side),
352: First outer pulley portion (first pulley, another pulley),
353: Second inner pulley portion (first pulley, another pulley),
354: Second outer pulley portion (first pulley, another pulley),
355: Third inner pulley portion (second pulley, shaft-side pulley),
355d: End portion of the third inner pulley (end portion on the opposite side), 356: Third outer pulley portion (second pulley, shaft-side pulley),
357: Fourth inner pulley portion (second pulley, shaft-side pulley),
358: Fourth outer pulley portion (second pulley, shaft-side pulley),
A1: First shaft,
A2: Second shaft (pulley shaft, another pulley shaft),
A3: Third shaft (another pulley shaft),
A4: Fourth shaft (pulley shaft),
B, C1, C2, C3: Center,
D1: First predetermined groove width (groove width of another pulley),
D2: Second predetermined groove width (groove width of another pulley),
D3: Third predetermined groove width (groove width of shaft-side pulley),
D4: Fourth predetermined groove width (groove width of another pulley),
D5: Fifth predetermined groove width (groove width of shaft-side pulley),
F1: Straight line,
M1, M2: Gap,
R2: Rotation axis (Rotation center axis) of second shaft,
R3: Rotation axis (Rotation center axis) of third shaft,
R4: Rotation axis (Rotation center axis) of fourth shaft, and
W: Wire

## Claims

1. A robotic surgical instrument comprising:
an end effector (41);
a first support body (45, 345) that supports the end effector rotatably about a first shaft (A1);
an elongate element (44, 344) for operating the end effector;
a second support body (46, 346) that supports the first support body rotatably about a second shaft (A2);
a first pulley (51, 52, 351, 352, 353, 354) that is provided rotatably about a third shaft at a position between the first shaft and the second shaft and to which the elongate element is suspended;
a second pulley (53, 54, 355, 356, 357, 358) that is provided rotatably about the second shaft and to which the elongate element is suspended; and
a shaft (42) having an extending direction and connected to the second support body at an end portion of the shaft in the extending direction,
**characterized in that**
a groove width (D3, D5) of a shaft-side pulley (55, 56, 355, 356, 357, 358), which is a pulley that is provided at a position closest to the shaft among a plurality of pulleys including the first pulley and the second pulley and to which the elongate element is suspended, is greater than groove widths (D1, D2, D4) of the other pulleys (51, 52. 53, 54, 351, 352, 353, 354) among the plurality of pulleys.

2. The robotic surgical instrument according to claim 1, wherein
the groove width of the shaft-side pulley is 1.2 times or more and 2.0 times or less of the groove widths of the other pulleys.

3. The robotic surgical instrument according to claim 1 or 2, wherein
a center (C2, C3) of the groove of the shaft-side pulley in an axial direction of the second shaft is arranged closer to a center (C1, C2) of the second support body in the axial direction of the second shaft than a center (B) of the groove of each of the other pulleys in the axial direction of the second shaft.

4. The robotic surgical instrument according to any one of claims 1 to 3, wherein
a position of an end portion (53d, 355d) of the shaft-side pulley on the side opposite to a center of the second support body in the axial direction and a position of an end portion (51d, 52d, 351d, 355d) of each of the other pulleys on the side opposite to the center of the second support body in the axial direction are substantially the same as each other, when viewed along the extending direction of the shaft.

5. The robotic surgical instrument according to any one of claims 1 to 4, wherein
a position of a rotation center axis of the shaft-side pulley and a position of a rotation center axis of each of the other pulleys are arranged on a straight line (F1) extending along the extending direction of the shaft, when viewed along the axial direction of the second shaft.

6. The robotic surgical instrument according to any one of claims 1 to 5, wherein
the shaft-side pulley includes a shaft insertion hole (55c, 355c) through which a pulley shaft (A2, A4) is inserted,
each of the other pulleys includes another shaft insertion hole (253c, 351c) through which another pulley shaft (A1, A2) is inserted, and
a gap (M1) between said another shaft insertion hole and said another pulley shaft of one of the other pulleys that is adjacent to the shaft-side pully is greater than a gap (M2) between the shaft insertion hole and the pulley shaft of the shaft-side pulley.

7. The robotic surgical instrument according to any one of claims 1 to 6, wherein
the elongate element includes a wire (W),
the second support body includes a communication hole (8a) that penetrates, in the extending direction of the shaft, an end surface portion on the opposite side of the shaft, and
the surgical instrument further includes a seal member (47, 347) which seals the communication hole (171, 172) and includes an insertion hole through which the wire of the elongate element is inserted.

8. The robotic surgical instrument according to claim 7, wherein
the seal member is compressed between the second support body and the shaft.

9. The robotic surgical instrument according to any one of claims 1 to 8, wherein
the shaft-side pulley comprises a third pulley (55, 56) which is provided rotatably about a fourth shaft (A4) at a position between the second shaft and the shaft and to which the elongate element is suspended, and
the third pulley includes the groove width (D3) greater than those of the first pulley and the second pulley.

10. The robotic surgical instrument according to any one of claims 1 to 8, wherein
the shaft-side pulley comprises the second pulley, and
the second pulley includes the groove width (D5) greater than that of the first pulley.

11. The robotic surgical instrument according to claim 10, wherein
the second pulley comprises a plurality of second pulleys arranged side by side in the axial direction of the second shaft, and
a second pulley (355, 357) among the plurality of second pulleys that is arranged on a side of the center of the first support body in the axial direction of the second shaft includes the groove width (D5) greater than that of the first pulley.

12. The robotic surgical instrument according to any one of claims 1 to 11, wherein
the end effector includes one or two end effector members (6, 306).

## Patentansprüche

1. Robotergesteuertes chirurgisches Instrument, aufweisend:
einen Endeffektor (41);
einen ersten Trägerkörper (45, 345), der den Endeffektor drehbar um eine erste Welle (A1) trägt;
ein langgestrecktes Element (44, 344), um den Endeffektor zu betätigen;
einen zweiten Trägerkörper (46, 346), der den ersten Trägerkörper drehbar um eine zweite Welle (A2) trägt;
eine erste Riemenscheibe (51, 52, 351, 352, 353, 354), die an einer Position zwischen der ersten Welle und der zweiten Welle drehbar um eine dritte Welle vorgesehen ist und an der das langgestreckte Element aufgehängt ist;
eine zweite Riemenscheibe (53, 54, 355, 356, 357, 358), die drehbar um die zweite Welle vorgesehen ist und an der das langgestreckte Element aufgehängt ist; und
einen Schaft (42), der eine Erstreckungsrichtung aufweist und an einem Endteil des Schafts in der Erstreckungsrichtung mit dem zweiten Trägerkörper verbunden ist,
**dadurch gekennzeichnet, dass**
eine Rillenbreite (D3; D5) einer schaftseitigen Riemenscheibe (55, 56, 355, 356, 357, 358), welche eine Riemenscheibe ist, die unter einer Vielzahl von Riemenscheiben, die die erste Riemenscheibe und die zweite Riemenscheibe umfasst, an einer Position vorgesehen ist, die dem Schaft am nächsten liegt, und an der das langgestreckte Element aufgehängt ist, größer ist als Rillenbreiten (D1, D2, D4) der anderen Riemenscheiben (51, 52, 53, 54, 351, 352, 353, 354) unter der Vielzahl von Riemenscheiben.

2. Robotergesteuertes chirurgisches Instrument nach Anspruch 1, wobei
die Rillenbreite der schaftseitigen Riemenscheibe das 1,2-Fache oder mehr und das 2,0-Fache oder weniger der Rillenbreiten der anderen Riemenscheiben ist.

3. Robotergesteuertes chirurgisches Instrument nach Anspruch 1 oder 2, wobei
eine Mitte (C3) der Rille der schaftseitigen Riemenscheibe in einer axialen Richtung der zweiten Welle näher an einer Mitte (B) des zweiten Trägerkörpers in der axialen Richtung der zweiten Welle als eine Mitte (C1, C2) der Rille von jeder der anderen Riemenscheiben in der axialen Richtung der zweiten Welle angeordnet ist.

4. Robotergesteuertes chirurgisches Instrument nach einem der Ansprüche 1 bis 3, wobei
eine Position eines Endteils (53d, 355d) der schaftseitigen Riemenscheibe auf der einer Mitte des zweiten Trägerkörpers in der axialen Richtung entgegengesetzten Seite und eine Position eines Endteils (51d, 52d, 351d, 355d) von jeder der anderen Riemenscheiben auf der der Mitte des zweiten Trägerkörpers in der axialen Richtung entgegengesetzten Seite entlang der Erstreckungsrichtung des Schafts betrachtet im Wesentlichen identisch sind.

5. Robotergesteuertes chirurgisches Instrument nach einem der Ansprüche 1 bis 4, wobei
eine Position einer Drehachse der schaftseitigen Riemenscheibe und eine Position einer Drehachse von jeder der anderen Riemenscheiben auf einer entlang der Erstreckungsrichtung des Schafts verlaufenden geraden Linie (F1) entlang der axialen Richtung des Schafts betrachtet angeordnet sind.

6. Robotergesteuertes chirurgisches Instrument nach einem der Ansprüche 1 bis 5, wobei
die schaftseitige Riemenscheibe ein Welleneinsetzloch (55c, 355c) aufweist, durch das eine Riemenscheibenwelle (A2, A4) eingesetzt ist,
jede der anderen Riemenscheiben ein anderes Welleneinsetzloch (253c, 351c) aufweist, durch das eine andere Riemenscheibenwelle (A1, A2) eingesetzt ist, und
ein Spalt (M1) zwischen dem anderen Welleneinsetzloch und der anderen Riemenscheibenwelle von einer der anderen Riemenscheiben, die der schaftseitigen Riemenscheibe benachbart ist, größer ist als ein Spalt (M2) zwischen dem Welleneinsetzloch und der Riemenscheibenwelle der schaftseitigen Riemenscheibe.

7. Robotergesteuertes chirurgisches Instrument nach einem der Ansprüche 1 bis 6, wobei
das langgestreckte Element einen Draht (W) umfasst,
der zweite Trägerkörper ein Verbindungsloch (8a) aufweist, das in der Erstreckungsrichtung des Schafts einen Endflächenteil auf der entgegengesetzten Seite des Schafts durchdringt und
das chirurgische Instrument ferner ein Dichtungselement (47, 347) enthält, das das Verbindungsloch (171, 172) abdichtet und ein Einsetzloch aufweist, durch das der Draht des langgestreckten Elements eingeführt ist.

8. Robotergesteuertes chirurgisches Instrument nach Anspruch 7, wobei
das Dichtungselement zwischen dem zweiten Trägerkörper und dem Schaft komprimiert ist.

9. Robotergesteuertes chirurgisches Instrument nach einem der Ansprüche 1 bis 8, wobei
die schaftseitige Riemenscheibe eine dritte Riemenscheibe (55, 56) aufweist, die an einer Position zwischen der zweiten Welle und dem Schaft drehbar um eine vierte Welle (A4) vorgesehen ist und an der das langgestreckte Element aufgehängt ist, und
die dritte Riemenscheibe die Rillenbreite (D3) aufweist, die größer als jene der ersten Riemenscheibe und der zweiten Riemenscheibe ist.

10. Robotergesteuertes chirurgisches Instrument nach einem der Ansprüche 1 bis 8, wobei
die schaftseitige Riemenscheibe die zweite Riemenscheibe aufweist und
die zweite Riemenscheibe die Rillenbreite (D5) aufweist, die größer als jene der ersten Riemenscheibe ist.

11. Robotergesteuertes chirurgisches Instrument nach Anspruch 10, wobei
die zweite Riemenscheibe eine Vielzahl zweiter Riemenscheiben aufweist, die in der axialen Richtung der zweiten Welle nebeneinander angeordnet sind, und
eine zweite Riemenscheibe (355, 357) unter der Vielzahl zweiter Riemenscheiben, die auf einer Seite der Mitte des ersten Trägerkörpers in der axialen Richtung der zweiten Welle angeordnet ist, die Rillenbreite (D5) aufweist, die größer als jene der ersten Riemenscheibe ist.

12. Robotergesteuertes chirurgisches Instrument nach einem der Ansprüche 1 bis 11, wobei
der Endeffektor ein oder zwei Endeffektorelemente (6, 306) umfasst.

## Revendications

1. Instrument chirurgical robotique comprenant :
un effecteur terminal (41) ;
un premier corps de support (45, 345) qui supporte l'effecteur terminal en rotation autour d'un premier arbre (A1) ;
un élément allongé (44, 344) pour actionner l'effecteur terminal ;
un deuxième corps de support (46, 346) qui supporte le premier corps de support en rotation autour d'un deuxième arbre (A2) ;
une première poulie (51, 52, 351, 352, 353, 354) qui est prévue en rotation autour d'un troisième arbre à une position entre le premier arbre et le deuxième arbre et à laquelle l'élément allongé est suspendu ;
une deuxième poulie (53, 54, 355, 356, 357, 358) qui est prévue en rotation autour du deuxième arbre et à laquelle l'élément allongé est suspendu ; et
un arbre (42) présentant une direction d'extension et connecté au deuxième corps de support à une partie d'extrémité de l'arbre dans la direction d'extension,
**caractérisé en ce que**
une largeur de rainures (D3, D5) d'une poulie côté arbre (55, 56, 355, 356, 357, 358), qui est une poulie prévue à une position la plus proche de l'arbre parmi une pluralité de poulies comportant la première poulie et la deuxième poulie et à laquelle l'élément allongé est suspendu, est supérieure à des largeurs de rainures (D1, D2, D4) des autres poulies (51, 52, 53, 54, 351, 352, 353, 354) parmi la pluralité de poulies.

2. Instrument chirurgical robotique selon la revendication 1, dans lequel
la largeur de rainure de la poulie côté arbre est 1,2 fois ou plus et 2,0 fois ou moins les largeurs de rainure des autres poulies.

3. Instrument chirurgical robotique selon la revendication 1 ou 2, dans lequel
un centre (C3) de la rainure de la poulie côté arbre dans une direction axiale du deuxième arbre est agencé plus près d'un centre (B) du deuxième corps de support dans la direction axiale du deuxième arbre qu'un centre (C1, C2) de la rainure de chacune des autres poulies dans la direction axiale du deuxième arbre.

4. Instrument chirurgical robotique selon l'une des revendications 1 à 3, dans lequel
une position d'une partie d'extrémité (53d, 355d) de la poulie côté arbre sur le côté opposé à un centre du deuxième corps de support dans la direction axiale et une position d'une partie d'extrémité (51d, 52d, 351d, 355d) de chacune des autres poulies sur le côté opposé au centre du deuxième corps de support dans la direction axiale sont sensiblement identiques l'une à l'autre, en vue dans la direction d'extension de l'arbre.

5. Instrument chirurgical robotique selon l'une des revendications 1 à 4, dans lequel
une position d'un axe de centre de rotation de la poulie côté arbre et une position d'un axe de centre de rotation de chacune des autres poulies sont agencées sur une ligne droite (F1) s'étendant dans la direction d'extension de l'arbre, en vue dans la direction axiale du deuxième arbre.

6. Instrument chirurgical robotique selon l'une des revendications 1 à 5, dans lequel
la poulie côté arbre comporte un trou d'insertion d'arbre (55c, 355c) à travers lequel un arbre de poulie (A2, A4) est inséré,
chacune des autres poulies comporte un autre trou d'insertion d'arbre (253c, 351c) à travers lequel un autre arbre de poulie (A1, A2) est inséré, et
un espacement (M1) entre ledit autre trou d'insertion d'arbre et ledit autre arbre de poulie de l'une des autres poulies qui est adjacente à la poulie côté arbre est plus grand qu'un espacement (M2) entre le trou d'insertion d'arbre et l'arbre de poulie de la poulie côté arbre.

7. Instrument chirurgical robotique selon l'une des revendications 1 à 6, dans lequel
l'élément allongé comporte un fil (W),
le deuxième corps de support comporte un trou de communication (8a) qui pénètre, dans la direction d'extension de l'arbre, une partie de surface d'extrémité sur le côté opposé de l'arbre, et
l'instrument chirurgical comporte en outre un organe de scellement (47, 347) qui scelle le trou de communication (171, 172) et comporte un trou d'insertion à travers lequel le fil de l'élément allongé est inséré.

8. Instrument chirurgical robotique selon la revendication 7, dans lequel
l'organe de scellement est compressé entre le deuxième corps de support et l'arbre.

9. Instrument chirurgical robotique selon l'une des revendications 1 à 8, dans lequel
la poulie côté arbre comprend une troisième poulie (55, 56) qui est prévue en rotation autour d'un quatrième arbre (A4) à une position entre le deuxième arbre et l'arbre et à laquelle l'élément allongé est suspendu, et
la troisième poulie comporte la largeur de rainure (D3) supérieure à celles de la première poulie et de la deuxième poulie.

10. Instrument chirurgical robotique selon l'une des revendications 1 à 8, dans lequel la poulie côté arbre comprend la deuxième poulie, et
la deuxième poulie comporte la largeur de rainure (D5) supérieure à celle de la première poulie.

11. Instrument chirurgical robotique selon la revendication 10, dans lequel
la deuxième poulie comprend une pluralité de deuxièmes poulies agencées côte à côte dans la direction axiale du deuxième arbre, et
une deuxième poulie (355, 357) parmi la pluralité de deuxièmes poulies qui est agencée sur un côté du centre du premier corps de support dans la direction axiale du deuxième arbre comporte la largeur de rainure (D5) supérieure à celle de la première poulie.

12. Instrument chirurgical robotique selon l'une des revendications 1 à 11, dans lequel
l'effecteur terminal comporte un ou deux organes d'effecteur terminal (6, 306).
